# EUROPEAN PATENT APPLICATION

(11) **EP 3 730 474 A1**
(43) Date of publication of application: **28.10.2020**
(21) Application number: 19170779.3
(22) Date of filing: 24.04.2019
(51) Int. Cl.: C07C 29/152, C07C 31/04

(54) **USE OF RENEWABLE ENERGY IN METHANOL SYNTHESIS**

(71) Applicant: SABIC Global Technologies B.V., 4612 PX Bergen op Zoom (NL)
(72) Inventor: WARD, Andrew Mark, 6160 GA Geleen (NL); STEVENSON, Scott, 6160 GA Geleen (NL); OPRINS, Arno Johannes Maria, 6160 GA Geleen (NL); ZHAO, Zhun, 6160 GA Geleen (NL); ABBOTT, Tim, 6160 GA Geleen (NL); LAWSON, Kenneth Francis, 6160 GA Geleen (NL); SCHROER, Joseph Wiliam, 6160 GA Geleen (NL); HUCKMAN, Michael Edward, 6160 GA Geleen (NL)
(74) Representative: Sabic Intellectual Property Group

(57) **Abstract**

A method of producing methanol comprising: (a) reforming a reformer feed to produce a reformer product comprising synthesis gas; (b) shifting the reformer product to produce a shifted product comprising carbon monoxide, carbon dioxide and hydrogen; (c) removing water from the reformer product or the shifted product to provide a dry syngas; (d) compressing the dry syngas to provide a compressed, dry syngas; (e) heating the compressed, dry syngas to provide a methanol synthesis feed; (f) synthesizing methanol from the methanol synthesis feed to provide a methanol synthesis product; and/or (g) purifying the methanol synthesis product to produce a purified methanol product, wherein a majority or greater than or equal to about 40, 50, 60, 70, 80, or 90% of the net energy needed in (a), (b), (c), (d), (e), (f), (g) or a combination thereof is provided by a non-carbon based energy source, a renewable energy source, and/or electricity.

## Description

### TECHNICAL FIELD

The present disclosure relates to the use of renewable energy in methanol synthesis; more particularly, the present disclosure relates to the electrification of a methanol synthesis plant; still more particularly, the present disclosure relates to a reduction in environmental emissions, such as carbon dioxide, by reducing the combustion of hydrocarbons (e.g., natural gas/fossil fuels) for fuel in a methanol synthesis plant.

### BACKGROUND

Chemical synthesis plants are utilized to provide a variety of chemicals. Often, a dedicated fuel is burned or 'combusted' to provide heat of reaction for chemical synthesis, energy to heat one or more process streams, energy to vaporize liquids (e.g., boil water used as a diluent), energy to do work (e.g., drive a compressor or pump), or energy for other process operations throughout the chemical synthesis plant. Such burning or combustion of fuels results in the production of flue gases, which can be harmful to the environment, and also results in a loss of energy efficiency of the process. Likewise, steam is often conventionally utilized as a plant-wide heat and/or energy transfer fluid within chemical synthesis plants. The steam utilized for the heat and/or energy transfer is often produced via the combustion of a fuel, resulting in the production of additional flue gas and further energy efficiency losses during the chemical synthesis. Additionally, the use of a material that could otherwise be utilized as a reactant for combustion as a fuel also reduces an amount of the desired chemical product produced in the chemical synthesis plant from a given amount of the material. Accordingly, a need exists for enhanced systems and methods of chemical synthesis whereby an amount of fuels, especially fossil fuels, burned to provide energy is reduced or eliminated. Desirably, such systems and methods also provide for an increase in energy efficiency and/or a decrease in emissions, such as emissions of greenhouse gases (GHG), by the chemical synthesis plant.

### SUMMARY

Herein disclosed is a method of producing methanol in a methanol synthesis plant, the method comprising: (a) subjecting a feed comprising methane, ethane, propane, butane, LPG, naphtha, biogas, or a combination thereof to reforming to produce a reformer product comprising carbon monoxide and hydrogen, wherein a reforming temperature is maintained by a net heat input Q1; (b) cooling the reformer product to produce a cooled reformer product by effecting a net heat removal Q2; (c) shifting the reformer product to produce a shifted reformer product; (d) cooling the shifted product by effecting a net heat removal Q3; (e) cooling and condensing water from the reformer product by effecting a net heat removal Q4 to provide a purified reformer product; (f) compressing the purified reformer product; (g) heating the compressed, purified reformer product by a net heat input Q5 to provide a methanol synthesis feed; (h) producing a product comprising methanol from the methanol synthesis feed; (i) cooling the product comprising methanol by a net heat removal Q6 to remove a crude methanol stream and provide a recycle gas stream comprising carbon monoxide and hydrogen; (j) compressing the recycle gas stream via a recycle compressor; (k) purging via a purge gas system, wherein purging may be effected by a net heat input or removal Q7; (1) purifying the methanol in the crude methanol product by removing light ends, heavy byproducts, water, or a combination thereof from the crude methanol stream, wherein removing the light ends, the heavy byproducts, the water, or the combination thereof requires a net heat input or removal Q8; or (m) a combination thereof, wherein, relative to a conventional method of producing methanol, more of the energy required for a net energy input or removal for a given step (a), (b), (c), (d), (e), (f), (g), (h), (i), (j), (k), (1), or a combination thereof (m), throughout the entire methanol synthesis plant, the compressing, or a combination thereof is provided by a non-carbon based energy source, a renewable energy source, and/or electricity.

Further disclosed herein is a method of producing methanol in a methanol synthesis plant, the method comprising: (a) subjecting a feed comprising methane, ethane, propane, butane, LPG, naphtha, biogas, or a combination thereof to reforming to produce a reformer product comprising carbon monoxide and hydrogen, wherein a reforming temperature is maintained by a net heat input Q1; (b) cooling the reformer product to produce a cooled reformer product by effecting a net heat removal Q2; (c) shifting the reformer product to produce a shifted reformer product; (d) cooling the shifted product by effecting a net heat removal Q3; (e) cooling and condensing water from the reformer product by effecting a net heat removal Q4 to provide a purified reformer product; (f) compressing the purified reformer product; (g) heating the compressed, purified reformer product by a net heat input Q5 to provide a methanol synthesis feed; (h) producing a product comprising methanol from the methanol synthesis feed; (i) cooling the product comprising methanol by a net heat removal Q6 to remove a crude methanol stream and provide a recycle gas stream comprising carbon monoxide and hydrogen; (j) compressing the recycle gas stream via a recycle compressor; (k) purging via a purge gas system, wherein purging is effected by a net heat input or removal Q7; (1) purifying the methanol in the crude methanol product by removing light ends, heavy byproducts, water, or a combination thereof from the crude methanol stream, wherein removing the light ends, the heavy byproducts, the water, or the combination thereof requires a net heat input or removal Q8; or (m) a combination thereof, wherein a majority of the energy required for a net energy input or removal for a given step (a), (b), (c), (d), (e), (f), (g), (h), (i), (j), (k), (1), or a combination thereof (m), throughout the entire methanol synthesis plant, the compressing, or a combination thereof is provided by a non-carbon based energy source, a renewable energy source, and/or electricity.

Also disclosed herein is a method of producing methanol, the method comprising: (a) reforming a reformer feed comprising methane, natural gas, ethane, propane, butane, naphtha, coal, or a combination thereof to produce a reformer product comprising synthesis gas; (b) optionally shifting the reformer product to produce a shifted product comprising carbon monoxide, carbon dioxide and hydrogen; (c) removing water from the reformer product or the shifted product to provide a dry syngas; (d) optionally compressing the dry syngas to provide a compressed, dry syngas; (e) heating the optionally compressed, dry syngas to provide a methanol synthesis feed; (f) synthesizing methanol from the methanol synthesis feed to provide a methanol synthesis product; and/or (g) purifying the methanol synthesis product to produce a purified methanol product, wherein a majority or greater than or equal to about 40, 50, 60, 70, 80, or 90% of the net energy needed in (a), (b), (c), (d), (e), (f), (g) or a combination thereof is provided by a non-carbon based energy source, a renewable energy source, electricity, or a combination thereof.

### DETAILED DESCRIPTION

As utilized herein, an 'intermittent energy source' or 'IES' is any source of energy that is not continuously available for conversion into electricity and outside direct control because the used energy cannot be stored or is economically undesirable. The availability of the intermittent energy source may be predictable or non-predictable. A renewable intermittent energy source is an intermittent energy source that is also a source of renewable energy, as defined hereinbelow. 'Intermittent electricity' refers to electricity produced from an IES.

As utilized herein 'renewable energy' and 'non-fossil based energy (E_{NF})' includes energy derived from a sustainable energy source that is replaced rapidly by a natural, ongoing process, and nuclear energy. Accordingly, the terms 'renewable energy' and 'non-fossil based energy (E_{NF})' refer to energy derived from a non-fossil fuel based energy source (e.g., energy not produced via the combustion of a fossil fuel such as coal or natural gas), while 'non-renewable' or 'fossil based energy (E_{F})' is energy derived from a fossil fuel-based energy source (e.g., energy produced via the combustion of a fossil fuel). Fossil fuels are natural fuels, such as coal or gas, formed in the geological past from the remains of living organisms. Accordingly, as utilized herein, 'renewable' and 'non-fossil based energy (E_{NF})' include, without limitation, wind, solar power, water flow/movement, or biomass, that is not depleted when used, as opposed to 'non-renewable' energy from a source, such as fossil fuels, that is depleted when used. Renewable energy thus excludes fossil fuel based energy (E_{F}) and includes biofuels.

As utilized herein, non-carbon based energy (E_{NC}) is energy from a non-carbon based energy source (e.g., energy not produced via the combustion of a carbon-based fuel such as a hydrocarbon), while carbon based energy (Ec) is energy from a carbon-based energy source (e.g., energy produced via the combustion of a carbon-based fuel such as a hydrocarbon). Nuclear energy is considered herein a renewable, non-fossil (E_{NF}) based energy and a non-carbon based energy (E_{NC}). Carbon-based energy (E_{C}) can thus be renewable (e.g., non-fossil fuel based) or non-renewable (e.g., fossil fuel-based). For example, various carbon-based biofuels are herein considered renewable, carbon-based energy sources.

As utilized herein 'renewable electricity' indicates electricity produced from a renewable energy source, while 'non-renewable electricity' is electricity produced from a non-renewable energy source. As utilized herein 'non-carbon based electricity' indicates electricity produced from a non-carbon based energy source, while 'carbon-based electricity' is electricity produced from a carbon-based energy source.

For example, in embodiments, renewable electricity and/or heat throughout the herein-disclosed methanol synthesis plant can be provided by the combustion of renewable hydrocarbons that come from renewable (e.g., biological) sources. For example, renewable electricity can, in embodiments, be produced via the combustion of an E_{NF}/E_{C} energy source comprising methane produced in a digester fed with agricultural wastes. Likewise, in embodiments, an E_{NF}/E_{C} energy source comprising synthesis gas produced using short cycle carbon waste materials can be utilized as a fuel (e.g., combusted to produce renewable electricity and/or heat). Desirably, the carbon dioxide generated by such combustion is recaptured (e.g., by the growth of a new crop).

As utilized 'externally' combusting a fuel refers to combusting a fuel outside of a reactor, e.g., in a furnace. Combustion as a part of the primary reaction (e.g., combustion which takes place with reforming in autothermal reforming (ATR)) would not be considered 'externally' combusting. As utilized herein, a 'dedicated' fuel is a fuel or portion of a feed stream introduced solely to provide fuel value (e.g., combustion heat) and not be converted into product.

As utilized herein, heat transfer steam (S_{HT}) indicates steam produced solely or primarily as an energy or heat transfer medium (e.g., steam not utilized as a diluent and/or reactant).

As utilized herein, 'net' heat input or removal refers to heat input or removal that results in primary energy consumption, e.g., heat input or removal not provided from another section or stream of the plant, e.g., not provided via heat exchange with another process stream. Similarly, 'net' energy refers to energy that results in primary energy consumption, e.g., energy not provided from another section or stream of the plant, e.g., thermal energy not provided via heat exchange with another process stream.

Although the majority of the above definitions are substantially as understood by those of skill in the art, one or more of the above definitions can be defined hereinabove in a manner differing from the meaning as ordinarily understood by those of skill in the art, due to the particular description herein of the presently disclosed subject matter.

The goal of a typical traditional chemical process is to convert feed A into product B, although often some byproducts (indicated as stream C) are also produced.

The unit operations used to effect this transformation require significant amounts of energy. Conventionally, this energy is primarily supplied by burning a fuel, often natural gas, to generate heat, denoted as ΔH_{c} (e.g., heat of combustion). This results in the undesirable production and emission of carbon dioxide (CO₂). Additional energy may be supplied by the heat of reaction, ΔHᵣ, if the reaction is exothermic; if the reaction is endothermic, an additional amount of energy equal to ΔHᵣ will need to be added. The total energy balance may also be affected if some byproducts are burned to produce energy, indicated as ΔH_{bp}. However, many chemical processes, even those involving exothermic reactions, are net energy consumers and thus require an external source of energy (typically provided by a hydrocarbon fuel(s)) to provide net process energy.

Electricity is usually only a small external input into most chemical production processes. Internal electrical requirements, such as for lighting or control, are usually so small as to be negligible, and in those few processes which require large amounts of electricity, for example, electrochemical reactors (e.g., the chlor-alkali process to make chlorine (Cl₂) and sodium hydroxide (NaOH)), this electricity is commonly generated within the plant boundaries by the combustion of hydrocarbons, and, even when not generated within the plant boundaries, if the electricity is obtained by the combustion of hydrocarbons rather than renewably, such use of electricity is equivalent in terms of energy efficiency and CO₂ emissions to on-site production of the electricity via hydrocarbon combustion.

Within most chemical production processes, energy consumption can conveniently be divided into three main categories. In the first such broad category, referred to herein as first category C1, heat is supplied directly as thermal energy by the combustion of a fuel (e.g., natural gas/fossil fuels) in a furnace. (As utilized, here, 'directly' indicates the absence of an intermediate heat transfer medium, such as steam.) These furnaces are often operated at high temperature and require large heat fluxes. The energy efficiency of such furnaces is limited by the heat losses in the furnace flue gas. Even where these heat losses are minimized by the cooling of the flue gas to recover energy, for example to generate steam or provide process heating, the conversion of the chemical energy contained in the fuel to usable thermal energy generally does not exceed 85 to 90%, even with substantial investment and loss of design and operating flexibility.

The second broad category, referred to herein as second category C2, of energy consumption in chemical processes comprises the heating of various chemical streams, primarily either to raise the temperature thereof to a desired reaction temperature or to provide energy for separations, most commonly distillation. Although some of this heat can be obtained by exchange with other chemical streams, it is most typically provided either by steam generated directly by the combustion of hydrocarbon fuels (e.g., natural gas/fossil fuels) or by heat transfer from the flue gas from high-temperature furnaces (e.g., from category C1). Most modern chemical processes include a relatively complicated steam system (or other heat transfer fluid system which will generically be referred to herein for simplicity as a steam heat transfer system) to move energy from where it is in excess to where it is needed. This steam system may include multiple pressure levels of steam to provide heat at different temperatures, as well as a steam and condensate recovery system, and is subject to corrosion, fouling, and other operational difficulties, including water treatment and contaminated condensate disposal. The fraction of the energy contained in the steam that can be used to heat process streams is generally limited to 90 to 95% by practical constraints on heat transfer, steam condensation, and boiler water recycle. If the steam was generated by an on-purpose external boiler, at most 80 to 85% of the chemical energy contained in the fuel will be used as heat by the chemical process, since an additional 10 to 15% or more will be lost to flue gas as in first category C1.

The third major category, referred to herein as third category C3, of energy usage in chemical processes is energy utilized to perform mechanical work. This work is primarily utilized for pressurizing and moving fluids from one place to another, and is used to drive rotating equipment such as pumps, compressors, and fans. This third category C3 also includes refrigeration equipment, since it is primarily powered by compression. In most chemical facilities, the energy for this work is provided by steam, obtained either by heat transfer with hot process streams, by heat transfer with partially-cooled flue gas streams from a furnace (e.g., in the convection section) in category C1, or directly from the combustion of hydrocarbons (e.g., natural gas/fossil fuels) in an on-purpose external boiler. Because of limitations on the conversion of thermal energy to mechanical work, the energy efficiency of these uses relative to the contained chemical energy of the hydrocarbons used as fuel is low, typically only 25 to 40%.

It has been unexpectedly discovered that using electricity (e.g., renewable and/or non-renewable electricity) to replace energy obtained from a hydrocarbon fuel in a chemical process can improve the process by increasing overall energy efficiency, while decreasing carbon dioxide emissions. In some cases, using electricity (e.g., renewable and/or non-renewable electricity) to replace energy obtained from a hydrocarbon fuel in a chemical process can also improve reliability and operability, decrease emissions of, for example, NOx, SOx, CO, and/or volatile organic compounds, and/or decrease production costs (e.g., if low-cost electricity is available).

According to embodiments of this disclosure, heat conventionally supplied as thermal energy by the combustion of a fuel (e.g., natural gas/fossil fuels) in a furnace and/or other heating in first category C1 is replaced by electrical heating. Electrical heat, electrical heating, generating heat electrically, electrical heater apparatus, and the like refer to the conversion of electricity into thermal energy available to be applied to a fluid. Such electrical heating includes, without limitation, heating by impedance (e.g., where electricity flows through a conduit carrying the fluid to be heated), heating via ohmic heating, plasma, electric arc, radio frequency (RF), infrared (IR), UV, and/or microwaves, heating by passage over a resistively heated element, heating by radiation from an electrically-heated element, heating by induction (e.g., an oscillating magnetic field), heating by mechanical means (e.g. compression) driven by electricity, heating via heat pump, heating by passing a relatively hot inert gas or another medium over tubes containing a fluid to be heated, wherein the hot inert gas or the another medium is heated electrically, or heating by some combination of these or the like.

According to embodiments of this disclosure, the utilization of steam (or another heat transfer fluid) as in second category C2 is eliminated and/or any steam (or other fluid) utilized solely as an intermediate heat transfer medium is electrically produced or heated (e.g., via electrical heating of water).

According to embodiments of this disclosure, conventional rotating equipment (e.g., steam turbines) utilized in third category C3 is replaced with electrically driven apparatus. According to embodiments of this disclosure, heat removal in third category C3 is replaced by electrically-powered heat removal, e.g., cooling and/or refrigeration. Electrical cooling, electrical coolers, removing heat electrically, electrical cooling or refrigeration apparatus, and the like refer to the removal of thermal energy from a fluid. Such electrical cooling includes, without limitation, cooling by electrically powered apparatus. For example, and without limitation, electrical cooling can be provided by powering a refrigeration cycle with electricity, wherein a refrigerant is compressed by an electrically powered compressor. As another example, electrical cooling can be provided by powering a cooling fan that blows air, wherein the air cools a process fluid or element. In embodiments, electrical heating and cooling can be effected by any electrical source.

A process driven by renewable energy can, in embodiments, appear similar to a conventional chemical process. However, a portion, a majority, or, in some cases, substantially all of the energy input supplied by fuel can be replaced by renewable energy and/or by renewable electricity. Such replacement of fuel input by non-carbon based energy, renewable energy, and/or renewable electricity will allow for a significant decrease in CO₂ emissions, in embodiments. In embodiments, any available form of renewable energy can be employed. However, the gains may be greatest if renewable electricity is utilized. The renewable energy can be obtained from, for example and without limitation, solar power, wind power, or hydroelectric power. Other types of renewable energy can also be applied in chemical plants according to embodiments of this disclosure. For example, in embodiments, concentrated solar power, geothermal energy, and/or the use of direct solar heating can be used to provide thermal energy and to decrease CO₂ emissions.

One of the main advantages to supplying needed energy via (e.g., renewable) electricity can be that the energy efficiency of the process will increase. Table 1 shows the energy efficiency of unit operations exemplifying the three categories of energy use in a chemical plant described above as C1, C2, and C3. It can be seen from Table 1 that the efficiency of each of the three categories of energy consumption is greater when electrical power is used. The gain can be greatest when steam drives for rotating equipment are replaced, according to embodiments of this disclosure, with electrical motors (as in third category C3, discussed hereinabove), which can operate with as much as three times the energy efficiency of steam drives. These gains are only realized when the electricity is derived from non-carbon based renewable sources, since the generation of electricity from carbon-based fuel combustion is only 30 to 45% energy efficient. Energy efficiency gains when using renewable electricity for heating applications (as in first category C1 and second category C2, discussed hereinabove) are smaller, but still significant. The net result is that less total energy will be used if renewable energy is used in place of carbon-based fuels (e.g., natural gas or other hydrocarbons).

| **Table 1: Energy Efficiency of Unit Operations** | | |
|---|---|---|
| Use | Efficiency from Hydrocarbon Combustion | Efficiency from Electricity According to This Disclosure |
| C1: Direct Heating | up to 80 - 90% | 95+% |
| C2: Heating with Steam | up to 80 - 95% | 95+% |
| C3: Rotating Equipment | 25 - 40% | 90 - 95% |

According to this disclosure, non-carbon based energy, renewable energy, and/or electricity (e.g., from renewable and/or non-renewable sources) can be utilized rather than conventional energy sources in categories C1, C2, and/or C3 described hereinabove. In embodiments, electrification is utilized for a majority of or substantially all utilities. In embodiments, electrification is utilized for a majority of or substantially all unit operations. In embodiments, electrification is utilized for a majority of or substantially all utilities and unit operations. In embodiments, electrification is utilized for a majority of or substantially all process applications, engines, cooling and/or heating (e.g., electrically driven heat pumps, refrigeration, electrical heating), radiation, storage systems, or a combination thereof.

In embodiments, the non-carbon based and/or renewable energy source comprises wind, solar, geothermal, hydroelectric, nuclear, tide, wave, ocean thermal gradient power, pressure-retarded osmosis, or a combination thereof. In embodiments, the non-carbon based energy source comprises hydrogen. In embodiments, electricity for electrification as described herein is produced from such a renewable and/or non-carbon based energy source. In embodiments, some or all of the electricity is from a non-renewable and/or carbon-based source, such as, without limitation, combustion of hydrocarbons (*e.g*., renewable or non-renewable hydrocarbons), coal, or hydrogen derived from hydrocarbons (*e.g*., renewable or non-renewable hydrocarbons).

The majority of the CO₂ emitted from most chemical plants is a result of fossil fuel combustion to provide energy for the plant. An additional benefit of using renewable energy in chemical synthesis as per embodiments of this disclosure is that the amount of greenhouse gases emitted will be significantly (e.g., by greater than or equal to at least 5, 10, 20, 30, 40, 50, 60, 70, 80, 90, or 100%) reduced relative to an equivalent conventional chemical synthesis plant or method in which hydrocarbons and/or fossil fuel(s) may be combusted. The burning of hydrocarbons (e.g., natural gas, methane) to generate energy results in the production of carbon dioxide (CO₂); this production can be reduced or avoided by the use of renewable energy according to embodiments of this disclosure. In embodiments of this disclosure, the amount of CO₂ produced per ton of product produced is reduced to less than or equal to about 1.6, 1.5, 1.4, 1.3, 1.2, 1.1, 1.0, 0.75, 0.5, 0.30, 0.25, 0.2, 0.1, 0.05, or 0 tons CO₂ per ton chemical product (e.g., methanol). Furthermore, in embodiments of this disclosure, the use of renewable energy frees up these hydrocarbons (e.g., natural gas, methane) typically burned for fuel for use as a chemical feedstock (e.g., to make methanol), which is a higher value use.

The use of renewable electricity in the production of chemicals can also lead to operational advantages. For example, in embodiments, electric power can be utilized to provide a more accurate and tunable input of heat, for example to control the temperature profile along a reactor or to change the temperature of specific trays in a distillation column. In embodiments, the use of electric heating in a reaction section (e.g., in a pyrolysis reaction section) leads to better controlled decoking and/or faster decoking. Without limitation, other examples include the use of electric powered refrigeration units to increase the efficiency of separations, and the replacement of inefficient stand-by gas-fired boilers with quick-acting on-demand electrical heaters and steam generators and for other utility uses. The use of electricity may also allow for significant operational advantages during start-up or shut-down, or to respond to process variability. In general, electricity as an energy source can be applied in specific locations and in precise and tunable amounts with a rapid response to process changes, leading to a variety of advantages over the use of thermal/combustion energy.

The use of renewable electricity according to embodiments of this disclosure can also increase the energy efficiency of utilities that supply energy to more than one chemical plant (e.g., a methanol synthesis plant and a nearby ammonia synthesis plant or a methanol synthesis plant and a nearby olefin synthesis plant). For example, if the compressors in an air separation unit that provides oxygen and nitrogen to several different production facilities are powered with renewable electricity, significant energy gains can be achieved relative to supplying this power with steam derived from the combustion of natural gas.

Energy recovery may be provided, in embodiments, via high temperature heat pumps or vapor recompression. The plant may further comprise heat and/or energy storage, for example, for use when an intermittent energy source (IES) is utilized. In embodiments, waste heat can be upgraded to usable temperature levels via electrically driven heat pumps. In other embodiments, energy can be recovered as electricity when process stream pressures are reduced by using a power-generating turbine instead of a control valve. In other embodiments, energy can be recovered as electricity using thermoelectric devices.

The use of renewable electricity to replace natural gas or other hydrocarbons as a source of energy, according to embodiments of this disclosure, can be done as part of a retrofit of an existing chemical process (e.g., an existing methanol synthesis plant) or as an integral component of the design of a new chemical plant (e.g., a new methanol synthesis plant). In a retrofit, opportunities for using renewable energy can depend on elements of the existing design, such as the steam system; in a retrofit, careful examination of the entire energy balance and steam system will be required, as electrifying individual pieces of equipment without regard to these considerations may result in energy inefficiencies. In embodiments, as seen in Table 1, the highest efficiency gains are achieved by replacing steam drives for rotating equipment (e.g., in third category C3) with electric motors. However, differing objectives may lead to different choices in partial electrification; in embodiments, in some instances greater CO₂ reductions at the expense of smaller increases in energy efficiency may sometimes be realized by first replacing hydrocarbon-fired furnaces (e.g., in first category C1). In embodiments, if thermal energy and/or steam are obtained from more than one hydrocarbon source, the most advantageous operation can be achieved by eliminating the most expensive and/or polluting fuel sources first. How much renewable energy can be included and to what extent existing fuel consumption and carbon dioxide (CO₂) emissions can be decreased can vary depending on the application, and will be within the skill of those of skill in the art upon reading this disclosure.

In embodiments, planning for the use of renewable energy in the design of a grass-roots chemical facility (e.g., a grass-roots methanol synthesis plant) can allow for more significant opportunities for better energy efficiency and lower CO₂ emissions. In embodiments, powering all rotating equipment (e.g., in third category C3) with electricity is utilized to realize large gains in energy efficiency. In embodiments, substantially all (or a majority, or greater than 40, 50, 60, 70, 80, or 90%) electric heating (e.g., in first category C1 and/or second category C2) is utilized, and the inefficiencies due to the loss of heat in flue gas are substantially reduced or even avoided. In embodiments, the use of steam generated via the combustion of a fossil fuel (e.g., in second category C2) can be minimized or avoided altogether. In embodiments, a change in catalyst and/or a modification of reactor operating conditions is utilized to allow for less heat generation in a reactor and/or the production of fewer byproducts that are burned. In embodiments, a plant (e.g., methanol synthesis plant) design based on the use of renewable electricity allows for enhanced optimization of separations operations, since the relative costs of compression and refrigeration are changed via utilization of renewable electricity as per this disclosure. Such enhanced separations can, in embodiments, also allow for further capture of minor byproducts from vent streams, freeing these minor products up for further use as feedstocks or products. Furthermore, the use of low-cost electricity, according to embodiments of this disclosure, may allow for the introduction of novel technologies such as, without limitation, hybrid gas and electric heaters, variable speed compressor drives, distributed refrigeration, heat pumps, improved distillation columns, passive solar heating of fluids, precise control of reactor temperature profiles, new materials of construction, and quench or cooling using electrically refrigerated diluents. If the cost of electricity is sufficiently low, utilization of such electricity as taught herein may favor the introduction of new electrochemical processes. For new construction, it may be less capital intensive to drive processes electrically, due, for example, to the lack of a (e.g., plant-wide) steam distribution system.

According to embodiments of this disclosure, non-carbon based energy, renewable energy, and/or electricity (renewable, non-renewable, carbon-based, and/or non-carbon based electricity) can be used in the production of nearly every chemical, including but not limited to methanol, ammonia, olefins (e.g., ethylene, propylene), aromatics, glycols, and polymers. Non-carbon based energy, renewable energy, and/or electricity can also be used, in embodiments, in the preparation of feedstocks for chemicals and for fuels production, such as in methyl tert-butyl ether (MTBE) synthesis, cracking, isomerization, and reforming. In such embodiments, some (e.g., at least about 10, 20, 30, 40, or 50%), a majority (e.g., at least about 50, 60, 70, 80, 90, or 95%), or all (e.g., about 100%) of the heating throughout the plant/process or a section thereof can be provided by electrical heating and/or some (e.g., at least about 10, 20, 30, 40, or 50%), a majority (e.g., at least about 50, 60, 70, 80, 90, or 95%), or all (e.g., about 100%) of the cooling throughout the plant/process or a section thereof can be provided by electrical cooling as described hereinabove. Disclosed hereinbelow is the use of renewable energy, non-carbon based energy, and/or electricity in methanol synthesis applications.

This disclosure describes a methanol synthesis plant for producing methanol, wherein the plant is configured such that a majority of the net energy required by one or more sections, units, or groups of like units or unit operations of the methanol synthesis plant is provided by non-carbon based energy (E_{NC}) from a non-carbon based energy source (e.g., not produced via the combustion of a carbon-based fuel such as a hydrocarbon), from renewable energy (e.g., from non-fossil fuel derived energy (E_{NF}) from a non-fossil fuel based energy source (e.g., not produced via the combustion of a fossil fuel such as coal or natural gas)), and/or from electricity. The E_{NC} or E_{NF} source may, in embodiments, comprise, primarily comprise, consist essentially of, or consist of electricity. The E_{NC} or E_{NF} source may, in embodiments, comprise, primarily comprise, consist essentially of, or consist of renewable electricity. In embodiments a portion (e.g., greater than or equal to about 5, 10, 20, 30, 40, 50),), a majority (e.g., greater than or equal to about 50, 60, 70, 80, 90, or 95%), or all (e.g., about 100%) of the net energy needed by the overall methanol synthesis plant, a section of the plant (e.g., a feed pretreating section, a synthesis gas (or 'syngas') production (e.g. reforming) section, a methanol synthesis section, and/or a methanol purification section), a group of like units (e.g., compressors, power providing units, heating units, reboilers, cooling units, refrigeration units, separators, reformers, methanol synthesis reactors, distillation/fractionation columns), or unit operations (e.g., compression, powering, heating operations, cooling operations, reforming operations, separations) of the plant, or a combination thereof is provided by electricity, renewable energy (e.g., non-fossil fuel derived energy (E_{NF})), and/or non-carbon based energy (E_{NC}). In embodiments, electricity is provided from a renewable energy source, such as, without limitation, wind (*e.g*., via wind turbines), solar (e.g., photovoltaic (PV) panels or solar thermal), hydroelectric, wave, geothermal, nuclear, tide, biomass combustion with associated capture of CO₂ in replacement crops, or a combination thereof. In embodiments a portion (e.g., greater than or equal to about 5, 10, 20, 30, 40, 50), a majority (e.g., greater than or equal to about 50, 60, 70, 80, 90, or 95%), or all (e.g., about 100%) of the electricity, renewable energy (e.g., non-fossil fuel derived energy (E_{NF})), or non-carbon based energy (E_{NC}) needed by the overall methanol synthesis plant, a section of the plant (e.g., a feed pretreating section, a syngas generation section, a methanol synthesis section, and/or a methanol purification section), a unit or a group of like units (e.g., compressors, power providing units, heating units, reboilers, cooling units, refrigeration units, reformers, methanol synthesis reactors, separators, distillation/fractionation columns) or unit operations (e.g., compressing, powering, separating, heating, cooling, reforming) of the methanol synthesis plant, or a combination thereof, and conventionally provided in a similar methanol synthesis plant via combustion of a fuel, a carbon-based fuel, and/or a fossil fuel and/or the use of steam (e.g., that was itself generated via the combustion of such a fuel) as an intermediate heat (and/or energy) transfer fluid, is provided without combusting a fuel, a carbon-base fuel, and/or a fossil fuel and/or without the use of steam generated by the combustion of such a fuel as an intermediate heat (and/or energy) transfer fluid. In embodiments, the net energy for the overall plant or one or more sections, units or groups of like units of the plant is provided by electricity from a renewable energy source. For example, in embodiments, heating is electrically provided via resistive heating or otherwise converting electrical energy into thermal and/or mechanical energy.

In embodiments, a methanol synthesis plant of this disclosure is configured such that a majority (e.g., greater than 50, 60, 70, 80, or 90%) of the net energy needed (e.g., in addition to heat exchange between process streams) for powering, heating, cooling, compressing, separating, or a combination thereof utilized via a feed pretreating system, one or more reformers, one or more methanol synthesis reactors, a methanol purification system, or a combination thereof, as described hereinbelow, is provided by electricity.

In embodiments, a methanol synthesis plant according to embodiments of this disclosure is a large plant having a production capacity for methanol of greater than or equal to about 200,000 tons per year, 1,000,000 tons per year, or 5,000,000 tons per year. At the larger sizes anticipated in this disclosure, the amount of energy provided by a non-carbon based energy source, a renewable energy source and/or electricity will be correspondingly large. In embodiments, a partially or completely electrified plant according to the methods of this disclosure will consume 20, 50, 100, 150, 200, 500, 750, or 1000 MW or more of electricity.

Although a specific embodiment of a methanol synthesis plant will be utilized to describe the electrification of a methanol synthesis plant, as disclosed herein, it is to be understood that numerous arrangements of units and a variety of methanol synthesis technologies utilizing a variety of feedstocks (e.g., natural gas, methane, LPG, naphtha, petcoke, coal, or a combination thereof) can be electrified as per this disclosure, as will be obvious to those of skill in the art upon reading the description herein. Although described at times hereinbelow with regard to the production of methanol via reforming of a feed comprising natural gas, methanol synthesis plants utilizing other technologies for the synthesis of methanol from a variety of feeds can be electrified as per this disclosure.

A methanol synthesis plant may be considered to include one or more of the following process sections for converting a feed stream 5 into a methanol product stream 45 (and optionally one or more byproduct streams 41): a feed pretreating section 10, a syngas synthesis section 20, a methanol synthesis section 30, a methanol purification section 40, or a combination thereof. Such sections will be described briefly in the next few paragraphs, and in more detail hereinbelow.

A feed pretreating section 10 of a methanol synthesis plant may be operable to prepare (e.g., remove undesirable components (e.g., sulfur) from, adjust temperature and/or pressure of a feed) a feed 5 for reforming, providing a pretreated feed 15. In applications, a methanol synthesis plant of this disclosure does not comprise a feed pretreating section. A syngas synthesis section 20 is operable to produce synthesis gas from feed 5 or pretreated feed 15 to produce a syngas synthesis product 25 comprising carbon monoxide (CO) and hydrogen (H₂). In embodiments, such as discussed further hereinbelow, syngas generation section 20 is a syngas synthesis section operable to carry out steam reforming of the feed (e.g., of a feed 5 or pretreated feed 15 comprising natural gas) to produce a reformer product comprising carbon monoxide (CO) and hydrogen (H₂). The syngas synthesis (or 'reformer') product 25 can further comprise carbon dioxide (CO₂), water, methane (CH₄), and/or impurities. A methanol synthesis section 30 is operable to produce methanol from the syngas synthesis product 25 and thus provide a crude methanol stream 35. A methanol purification section 40 is operable to separate a purified methanol product 45 and byproducts 41 from the crude methanol stream 35.

Energy (E) input to or within the methanol synthesis plant or one or more sections or groups of units, like units, or unit operations thereof (that may conventionally be provided via a carbon based energy (Ec) 2A from a carbon based energy source, a fossil fuel derived energy (E_{F}) 3A from a fossil fuel-based energy source, or via the use of steam (e.g., steam generated for this purpose using energy derived from a carbon or fossil fuel based energy source) solely or primarily as a heat or energy transfer medium (S_{HT}) 1), may be partially or completely replaced by non-carbon based energy (E_{NC}) 2B from a non-carbon based energy source, renewable/non-fossil fuel based energy (E_{NF}) 3B from a renewable energy source, and/or electricity (e.g., electricity and/or renewable electricity). The carbon based energy (E_{C}) 2A, the fossil fuel derived energy (E_{F}) 3A, or both can be partially or completely replaced by electricity. The electricity may be derived from a non-carbon based fuel, a renewable fuel, a renewable energy source, or a combination thereof, in embodiments. A benefit derived via the herein disclosed system and method may be a reduction in the greenhouse gas (GHG) emissions 4 from the methanol synthesis plant or process. The above-noted elimination or reduction of the steam system may also result in lower capital and operating costs, in embodiments.

According to this disclosure, when cooling process streams, as much heat as possible should be used to heat other process streams. However, below a certain temperature, further heat transfer is no longer effective or useful, and blowers, cooling water, and/or refrigeration (which require an energy input for heat removal) are utilized. In such embodiments, for example, heat exchangers, refrigeration units, or a combination thereof for altering the temperature of process streams may be powered electrically. In embodiments, steam is not utilized solely as an intermediate heat and/or energy transfer stream, and the plant or section(s) thereof do not comprise an elaborate steam system such as conventionally employed for energy transfer. In embodiments, steam is used as a heat transfer fluid and is not used to do mechanical work, for example to drive a pump or compressor. In embodiments, heating is provided via resistive heating. In embodiments, heating is provided via inductive heating.

Although not intending to be limited by the examples provided herein, a description of some of the ways a methanol synthesis plant can be electrified according to embodiments of this disclosure will now be provided with reference to the exemplary methanol synthesis block flow diagram of methanol synthesis plant II. The steps, sections, groups of units or unit operations described may be present or operated in any suitable order, one or more of the steps, sections, units, or unit operations may be absent, duplicated, replaced by a different step, section, unit, or unit operation, and additional steps, sections, units, or unit operations not described herein may be employed, in various embodiments. Additionally, although a step (e.g., syngas cooling B1) is noted as being in a particular section (e.g., in methanol synthesis section 30/130), the step could also be considered a part of another section (e.g., syngas synthesis (or 'reforming') section 20/120).

As noted hereinabove, in embodiments, a methanol synthesis plant of this disclosure comprises a feed pretreating section 10/110. Such a feed pretreating section 10/110 can be operable to remove one or more components such as, without limitation, sulfur and/or mercury, from a feed (e.g., a natural gas feed), adjust the pressure of the feed to a desired operating pressure, adjust the temperature of the feed to a desired operating temperature, pre-reform the feed, or a combination thereof.

As noted hereinabove, in embodiments, the pretreating section 10/110 is operable to provide the feed at a desired operating pressure for the downstream syngas synthesis section 20/120. In the exemplary methanol synthesis plant II, feed pretreating section 110 comprises apparatus for feed gas pressure adjustment C1, operable to adjust the pressure of a feed introduced thereto via natural gas feed stream 105. One or more compressors may be utilized to increase the pressure of the feed stream 105. In other embodiments, if the delivery pressure of the feed is higher than that required for the process, the pressure is lowered through a power-generating turbine such that the energy of the excess pressure is recovered, e.g. as electrical energy.

As noted hereinabove, in embodiments, the pretreating section 10/110 is operable to provide the feed at a desired operating temperature. For example, in the exemplary methanol synthesis plant II, feed pretreating section 110 comprises first feed preheating A1, second feed preheating A2, and third feed preheating A3, operable to adjust the temperature of a feed introduced thereto to a desired downstream temperature. The first feed preheating A1 may be configured to increase the temperature of the feed in stream 101 (via a first heat input Q1) to a desired temperature for feed purification at 110A (described below) in stream 102; second feed preheating A2 is configured to increase the temperature of the feed in stream 103 from feed purification 110A (via a second heat input Q2) to a desired temperature for feed pre-reforming at 110B (described below) in stream 104; third feed preheating A3 is configured to increase the temperature of the feed in stream 105 from feed pre-reforming (via a third heat input Q3) to a desired temperature in feed stream 115 for downstream reforming in downstream syngas synthesis section 120.

In embodiments, the heat obtained from cooling the reformer products (e.g., Q6, Q7, and/or Q8 discussed further hereinbelow) is used to preheat the feed (e.g., used to provide a first portion of the heat Q1, Q2, and/or Q3). In embodiments, a remainder of the heat needed for Q1, Q2, and/or Q3 is provided by resistive heating, superheated steam, internal oxidation obtained by adding a small amount of oxygen or air, or a combination thereof.

As noted hereinabove, in embodiments, the pretreating section 10/110 is operable to remove one or more components from a feed stream prior to downstream synthesis gas production in downstream syngas synthesis (e.g., reforming) section 20/120. For example, impurities such as sulfur compounds, carbon dioxide, nitrogen, mercury, or a combination thereof may be removed from a feed stream (e.g., a natural gas feed stream) by pretreating section 10/110. For example, if the feed comprises sulfur, sulfur compounds may be removed because sulfur deactivates the catalyst(s) used in subsequent steps. Sulfur removal can utilize catalytic hydrodesulfurization (HDS) to convert sulfur compounds in the feedstocks to gaseous hydrogen sulfide via the Equation (1):

H₂ + RSH → RH + H₂S (gas) (1).

The gaseous hydrogen sulfide can then be adsorbed and removed by passing it through beds of, for example, zinc oxide, where it is converted to solid zinc sulfide via the Equation (2):

H₂S + ZnO → ZnS + H₂O (2).

For example, in the exemplary methanol synthesis plant II, feed pretreating section 110 comprises feed purification (e.g., sulfur removal, Hg removal, etc.) 110A. Hydrogen stream 106 may be introduced into feed purification apparatus of feed purification (e.g., sulfur removal, Hg removal, etc.) 110A. Feed purification apparatus utilized in 110A can be any suitable contaminant/poison removal apparatus known to those of skill in the art.

As noted hereinabove, in embodiments, the pretreating section 10/110 is operable to pre-reform a feed. For example, in the exemplary methanol synthesis plant II, feed pretreating section 110 comprises feed pre-reforming 110B. Pre-reforming apparatus may be operable to remove higher hydrocarbons that may foul the main reformer. Feed pre-reforming apparatus utilized in 110B can be any suitable pre-reforming apparatus known to those of skill in the art.

According to embodiments of this disclosure, feed pretreating can be effected with a reduced usage of non-carbon based energy, non-fossil based energy, the use of renewable energy, or the use of electricity (e.g., electricity from renewable and/or non-renewable source(s)). For example: compressors of the pretreating section (e.g., feed gas pressure adjustment C1) can be operated with electric motors rather than gas or steam driven turbines, feed preheating A1, A2 and/or A3 can be effected with resistive heating, steam produced at steam generation 112 for pre-reforming (and/or downstream reforming or other syngas synthesis described below) can be generated electrically, feed purification apparatus at 110A and/or pre-reforming apparatus at 110B may be operated electrically, or a combination thereof. In embodiments, pretreatment beds can be regenerated using heat, steam, or other gases wherein the energy is supplied electrically. In embodiments, a feed preheater for a hydrodesulfurization reactor can use electrically powered heating. In embodiments, vapor compression heat pumps may be used to heat the feed and/or thermoelectric heaters and coolers may be used to heat or cool the feed. For example, steam generated by an electrode boiler, which converts over 99% of the input electrical energy into making steam, can be utilized to provide higher energy efficiency than a conventional hydrocarbon (or fossil fuel) fired steam boiler.

In embodiments, feed preheating can be done by electric resistive heating elements that can be arranged internally to a process conduit, within a heat transfer element sheath, and/or externally to the process conduit while heating the feed through the conduit. Heat from resistive heating elements may flow through heat transfer equipment (e.g. metal surfaces, fins, tubes, etc.) heat transfer gases, vapors, fluids, solids, plasmas, and/or other media and may transfer to the process in conductive, convective and/or radiative form. Ohmic resistive heating or inductive heating of the process conduits or equipment can be utilized, in embodiments. In embodiments, mechanical heating can be utilized. In embodiments, heating by electromagnetic waves, either by absorption of the process fluid or by absorption by another media that subsequently transfers heat to the process fluid can be utilized. In embodiments, vapor compression heat pumps, and/or thermoelectric heaters and/or coolers can be utilized. In embodiments, steam is generated using an electrode boiler or a resistive immersion heater. In embodiments, preheating is effected by the injection of a hot process fluid. In embodiments, this hot process fluid comprises steam. In embodiments, the steam is heated electrically. In embodiments, the steam is obtained from the oxidation of hydrogen or from fuel cell exhaust.

A methanol synthesis plant according to this disclosure comprises a syngas generation section operable to reform the hydrocarbon feed to produce hydrogen and carbon monoxide (e.g., synthesis gas or 'syngas'). Synthesis gas for the methanol synthesis may also or additionally be produced via partial oxidation (e.g. methane partial oxidation), and/or gasification (e.g. coal gasification), and such is intended to be within the scope of this disclosure.

Syngas synthesis section 20/120 can be or can include a reforming section, a partial oxidation section, a gasification section, or a combination thereof. In embodiments, syngas synthesis section 20/120 comprises reforming. Syngas synthesis section 120 can include steam methane reforming 120A, dry reforming 120B, autothermal reforming 120C, or a combination thereof. Optional technologies (not shown) include, without limitation, mixed reforming with steam, oxygen, and/or carbon dioxide. In embodiments, a reforming syngas synthesis section 120 comprises a steam methane reformer or reactor, a partial oxidation reformer or reactor, an autothermal reforming reactor, a dry reforming reactor, or a combination thereof. A heat input Q4 may be utilized to maintain a desired steam methane reforming temperature at 120A and/or a heat input Q5 may be utilized to maintain a desired dry reforming temperature at 120B. Carbon dioxide stream 123 may be utilized for dry reforming 120B and/or oxygen stream 128 may be utilized for autothermal reforming 120C. An air separation unit 127 may be utilized to provide oxygen stream 128 from air stream 126 for autothermal reforming 120C. Heat removal Q15 can be utilized to effect air separation in air separation unit(s) 127, for example cryogenic air separation unit(s), in embodiments. Steam stream 113 may be produced from water in water stream 111 via steam generation apparatus 112. The steam 113 can be utilized in a pre-reformer (when present) and a reformer (e.g., a steam methane reformer 120A).

A reforming section as syngas synthesis section 20/120 can be operable to effect catalytic steam reforming of the (e.g., sulfur-free) methane feed to form hydrogen plus carbon monoxide via Equation (3):

CH₄ + H₂O ⇄ CO + 3H₂ (3).

The water gas shift reaction shown in Equation (4) also occurs:

CO + H₂O ⇄ CO₂ + H₂. (4)

Generally, at equilibrium, the reactions of Equations (3) and (4) can be combined to provide the overall reaction of Equation (5):

CH₄ + 2H₂O ⇄ CO₂ +4H₂. (5)

For example, syngas synthesis section 120 may comprise steam methane reforming at 120A, whereby methane in methane feed 115 and steam in line 113 are combined and then fed to a steam methane reforming furnace of steam methane reforming 120A where the methane is partially converted to carbon monoxide and hydrogen via Equation (3). In embodiments, steam methane reforming (SMR) is effected at a temperature in a range of from about 500°C to about 1000 °C (about 930 °F to 1830 °F) and a pressure in a range of from about 8 to about 35 bars. The steam methane reforming (SMR) reaction is endothermic and the heat of reaction is conventionally provided by burning methane and/or process purge gases at furnace burners to provide heat input, indicated as Q4. As described further hereinbelow, according to embodiments of this disclosure, the heat input Q4 is provided via a renewable energy source and/or electricity. The renewable energy source can comprise electricity from a renewable energy source (such as wind or solar energy, etc.). Thus, according to embodiments of this disclosure, a steam methane reforming apparatus 120A is not heated via combustion of a dedicated fuel (e.g., is not produced via combustion of a carbon-based and/or fossil-based fuel in a furnace to provide heat for the reactions). Carbon dioxide produced via Equation (4) or (5) can be converted to methanol and thus not result in emissions conventionally seen when a dedicated fuel is combusted in a furnace to provide heat for the reactions and concomitantly produces a flue gas comprising CO₂.

In embodiments, a system or method of methanol synthesis according to this disclosure results in the production of less than or equal to about 2, 1.5, 1, 0.5, 0.1, or 0 (e.g., no) tons of a combustion flue gas produced via combustion of a fuel (e.g., a carbon-based fuel, a fossil fuel, or a combination thereof) per ton of methanol produced. In embodiments of this disclosure, the amount of CO₂ produced per ton of methanol produced is reduced to less than or equal to about 1.5, 1.2, 1.0, 0.75, 0.5, 0.30, 0.25, 0.2, 0.1, 0.05, or 0 tons CO₂ per ton chemical product (e.g., methanol). In embodiments, no flue gas is produced, for example, within the syngas synthesis section, one or more sections or all the sections of the methanol synthesis plant of this disclosure.

In embodiments, the steam methane reformer(s) is (are) heated electrically. In embodiments, the steam reformer(s) are heated with resistive or inductive heating. In embodiments, the steam reformer(s) are heated by means of radiative panels that are heated electrically (e.g., via resistive heating, inductive heating, ohmic heating, or the like.)

In embodiments, a 2-stage reformer is utilized, and oxygen 128 is added to the partially converted mixture from the first stage reformer wherein it combusts with methane to generate carbon oxides and heat via partial oxidation. For example, in embodiments, the temperature rises to a temperature in a range of from about 1200 to 1250°C at an inlet of an autothermal catalyst bed and to about 700 to 900°C at an exit of the autothermal catalyst bed). The combination of steam reforming and partial oxidation can be utilized, in embodiments, to control the composition of the resultant synthesis gas.

In embodiments, CO₂ stream 123 is utilized to add some CO₂ into the process gas stream either ahead of a syngas synthesis reactor (e.g., a steam reformer) or downstream of a syngas synthesis reactor (e.g., a steam reformer). This can allow a larger fraction of the hydrogen produced by the SMR to be incorporated into methanol molecules (e.g., by reaction with CO₂). In embodiments, CO₂ is introduced upstream of one or more methanol synthesis reactors of methanol synthesis section 30/130 (described further hereinbelow) to consume extra hydrogen, balance the heat in the one or more methanol synthesis reactors (e.g., make it less exothermic so that there is less required heat removal Q10), and allow for the electric conversion of CO₂ to methanol. Alternatively or additionally, CO₂ can be introduced, in embodiments, to a reverse water gas shift (WGS) reactor (e.g., to WGS 130A of methanol synthesis section 30/130, further described hereinbelow) to provide for endothermic and low-temperature WGS and/or an easy and low-temperature sink for electricity and/or excess low-grade heat from cooling product streams.

Syngas synthesis section 20/120 can comprise dry reforming. In dry reforming, reaction of methane and carbon dioxide produces synthesis gas in a process called dry reforming as no steam or less than normal quantities of steam utilized for Steam Methane Reforming is employed. Dry reforming proceeds as per Equation (6):

CH₄ + CO₂ ⇄ 2CO + 2H₂ ΔH_{298K}= 59.1 kcal/mol (6).

The reaction of Eq. (6) is more endothermic than steam methane reforming and produces a gas with significant hydrogen deficit for methanol synthesis. While this is a disadvantage for methanol synthesis, dry reforming may be utilized, in embodiments. In embodiments, dry reforming is utilized in conjunction with steam reforming.

The production of synthesis gas is generally strongly endothermic and requires the addition of thermal energy (provided as heat Q). As noted above, conventionally, a fuel (e.g., natural gas, methane, purge gas from the methanol synthesis section) is burned to provide heat (e.g., Q4 and/or Q5) needed to attain a desired reforming temperature and provide the necessary heat of reaction. In embodiments of this disclosure, a desired reforming temperature is attained without burning a fuel externally in a furnace. In embodiments of this disclosure, a desired reforming temperature is attained without burning a carbon-based fuel. In embodiments, a desired reforming temperature is achieved by the injection of steam produced electrically or from the combustion of hydrogen. Desirably, no fossil-based fuel, no carbon based fuel (e.g., methane, or natural gas), and/or no hydrogen is burned as a dedicated fuel in embodiments of this disclosure, as such materials conventionally burned as a fuel can then be utilized as a feed to produce additional methanol product according to embodiments of this disclosure.

In embodiments, syngas synthesis section 20/120 is configured for the production of carbon dioxide (CO₂) by combustion internally to the process to add heat to the syngas generation section and reduce heat generation in the one or more methanol synthesis reactors of a methanol synthesis section 30/130, described further hereinbelow. In embodiments, such carbon dioxide is produced via combustion of a renewable (e.g., a non-fossil) fuel.

In embodiments, the syngas synthesis section 20/120 is configured to supply syngas and/or purified hydrogen as a product. This can be done, for example, by cleaning contaminants by absorbing them in an absorbing solvent and then regenerating the solvent by electric heating. Alternately, pressure-swing or temperature swing adsorption could be employed in hydrogen separation and purification. Alternatively, membrane separations with electrically powered recycle or charge compressors could be used for the separation. Cryogenic distillation that is electrically driven could be employed in the purification. In other embodiments, the syngas or purified hydrogen product is an intermediate stream that is integrated with another chemical process that is the consumer of the hydrogen or syngas. In embodiments, the amount of hydrogen produced is increased by purposefully manipulating various process variables, e.g., steam to carbon ratio or oxygen to carbon ratio.

In embodiments, reforming can be effected with a reduced usage of non-carbon based energy (E_{NC}), the use of renewable energy (e.g., non-fossil fuel based energy (E_{NF})), or the use in electricity (e.g., electricity from renewable source(s)). For example: compressors and pumps of the syngas synthesis section (such as a compressor or pump for introducing steam 113, carbon dioxide 123, or oxygen 128) can be operated with electric motors or otherwise electrically-driven rather than via gas or steam driven turbines, the heat input Q4 or Q5 required to attain a desired reforming temperature (in a steam methane reformer 120A or a dry reformer 120B) can be electrically provided, or a combination thereof.

In embodiments, H₂O (e.g., water stream 111, CO₂ (e.g., CO₂ stream 123), O₂ (e.g., O₂ stream 128), or a combination thereof can be preheated electrically before introduction. In embodiments, steam for reforming is generated using an electrode boiler. In embodiments, H₂O and/or CO₂ (e.g., in water stream 111 and/or CO₂ stream 123), can be superheated electrically to raise the temperature of the process stream when they are introduced. Alternatively or additionally, in embodiments, the heat (e.g., Q3, Q4, and/or Q5) for the reforming reaction of a syngas synthesis section 20/120 can be provided electrically. In embodiments, reforming heat (e.g., Q3, Q4, and/or Q5) can be provided by electric resistive heating elements that can be arranged internally to a process conduit, within a heat transfer element sheath, and/or externally to the process conduit while heating the process through the conduit. Heat from resistive heating elements may flow through heat transfer equipment (e.g. metal surfaces, fins, tubes, etc.) heat transfer gases, vapors, fluids, solids, plasmas, and/or other media and may transfer to the process in conductive, convective or radiative form. Ohmic resistive heating or inductive heating of the process conduits or equipment may be utilized, in embodiments. In embodiments, mechanical heating can be utilized. Heating by electromagnetic waves, either by absorption of the process fluid or by absorption by another media that subsequently transfers heat to the process fluid may be utilized, in embodiments.

In embodiments, thermoelectric devices can be utilized to heat the reaction of syngas synthesis section 20/120 (e.g., steam reforming section 120A, dry reforming section 120B, and/or autothermal reforming section 120C) while providing cooling for the hot syngas. Multi-stage thermoelectric devices may be used to provide heat to the reaction coming from the downstream methanol synthesis reaction of methanol synthesis section 30/130 or another part of the process. Thermoelectric devices may be used to generate electricity while cooling the oxidation reaction (e.g., of a partial oxidation syngas synthesis section 20/120) or the reformer effluent (of a reforming syngas synthesis section 20/120). In cases where electric heating is used, one resulting advantage can be enhanced heat distribution and more heat uniformity. For example the tube metal temperature is a design limitation of a heater from a conventional combustion source, with the hottest part being located near the flame. By eliminating the flame, the design can be of an overall higher temperature on the process side, with more uniformity, potentially leading to higher conversions in the reformer and thus higher overall conversion. In embodiments, steam supplied to the syngas synthesis section 20/120 can be generated electrically. In embodiments, steam is used as a reactant and/or for dilution in syngas synthesis section 20/120, and the steam used for reactant and/or dilution is generated using electricity. In embodiments, oxygen supplied to the oxidative part of a reforming section of a syngas synthesis section 20/120 can be made by air separation powered by electricity. In embodiments, carbon dioxide supplied to dry or mixed reforming can be recovered and/or supplied by electrically heated and/or powered equipment.

In embodiments, at least 5%, 10%, 20%, 30%, 40%, 50%, 60%, 70%, 80%, 90%, a majority, or all of the air separation units (ASUs) of syngas synthesis section 20/120 or elsewhere in a methanol synthesis plant of this disclosure are electrically powered.

As noted above, a methanol synthesis plant of this disclosure can comprise a methanol synthesis section 30/130. After the reforming is complete, the syngas stream can be partially cooled, optionally fed to a water-gas shift reactor where additional hydrogen is formed by shifting water and carbon monoxide to produce additional hydrogen and carbon dioxide. The shifted stream can be cooled to near ambient temperature and much of the water condensed out to provide a largely dry gas called dry gas for the methanol synthesis. The pressure of the largely dry gas can be increased to a desired methanol synthesis reaction pressure, which can be, for example, from about 1000 to 3000 psia, via one or more compressors. Thus, in embodiments, the methanol synthesis section 30/130 can comprise cooling the syngas produced in the syngas synthesis section 20/120 at syngas cooling B1 and/or B2, water gas shifting the synthesis gas at 130A, condensing water at cooling B3, compressing the syngas at C2, heating the syngas at A4, forming methanol from the syngas at methanol reaction 130B, or a combination thereof. Methanol synthesis section 30/130 can thus comprise cooling apparatus to effect cooling B1/B2/B3 operable to remove heat from the syngas; water gas shift apparatus operable to subject the syngas to water-gas shifting at 130A, compression apparatus (also referred to herein as a syngas compressor) operable to compress the dry syngas at C2; heating apparatus operable to heat the syngas at syngas heating A4 for introduction into methanol synthesis at 130B; and/or methanol synthesis apparatus to carry out methanol synthesis at 130B.

As noted above, a methanol synthesis section 30/130 of this disclosure can comprise synthesis gas cooling B1/B2/B3. For example, after reforming in syngas synthesis section 20/120, the synthesis gas can be cooled. For example first syngas cooling B1 may be operable for heat removal (indicated at Q6) upstream of a water-gas shift 130A, second syngas cooling B2 may be operable for heat removal (indicated at Q7) downstream of a water-gas shift 130A, water condensing or third syngas cooling B3 may be operable for heat removal (indicated at Q8) and removing water 138 from the syngas upstream of methanol synthesis at 130B. As noted above, the methanol synthesis section 30/130 can comprise a water removal, "water condensing" or third cooling apparatus B3. The optionally shifted syngas stream 132 stream may be cooled in second syngas cooling B2 and the water 138 is condensed out (with heat removal indicated at Q8) at water condensing B3, to produce a dry gas stream 134.

As noted below, in embodiments, a majority, greater than 20, 30, 40, 50, 60, 70, 80, or 90%, or substantially all of the cooling B1/B2/B3 is powered by a non-carbon based energy source, e.g., electricity from a renewable energy source and/or cooling via heat exchange with the process stream in another section of the plant. For example, cooling can be at least partly achieved, in embodiments, by heat exchanging with the compressed syngas stream 136 (described hereinbelow) exiting syngas compression (e.g., using Q7 to provide some or all of the energy Q9). In embodiments, some or all of the heat recovered in cooling B1/B2/B3 is used in feed preheating A1/A2/A3. In embodiments, this is done using one or more feed/effluent heat exchangers.

As noted above, a methanol synthesis section 30/130 of this disclosure can comprise synthesis gas shifting 130A. For example, after reforming is complete, the reformer product stream 25/125 can be subjected to water gas shifting in a shift conversion section 130A to produce additional hydrogen via the water gas shift (WGS) reaction of Equation (4) above.

Desirably, and without limitation, reforming and/or shifting are operated, in embodiments, to provide a synthesis gas (*e.g*., for feeding to the methanol synthesis reactors of methanol synthesis 130B) that has a ratio of hydrogen to carbon monoxide (H₂/CO) of about 2 and a (H₂-CO₂)/(CO+CO₂) ratio, or stoichiometric number, equal to or slightly greater than 2. The shifting can be effected via any suitable methods known in the art, in embodiments. For example, shifting can comprise high temperature shift, low temperature shift, or both. The reformer product in reformer product stream 125 may be partially cooled at first cooling step or unit(s) B1 (with heat removal indicated by Q6) to produce a cooled reformer product indicated by stream 131. Cooled reformer stream 131 is introduced into shift reactor(s) at 130A where additional hydrogen (H₂) is formed by shifting H₂O and CO to produce CO₂ and additional hydrogen via the water gas shift (WGS) reaction of Equation (4) above to provide shifted stream 132. Cooling B2 of the shifted stream 132 may be performed in second cooling step or unit(s) B2 (with heat removal indicated by Q7) and water removal via cooling B3 (with heat removal indicated by Q8), whereby the stream is further cooled to provide cooled, shifted streams 133 and 134, respectively. Without limitation, a methanol synthesis plant of this disclosure may comprise both high temperature shift and low temperature shift at 130A because low temperature is needed to drive the reaction to near completion, but the reaction proceeds faster at high temperature.

In embodiments, a shift reactor is utilized downstream of the methanol synthesis reaction, for example, downstream of crude methanol recovery 140A and upstream of purge gas system 140B. In such embodiments, the shift reactor can be utilized to shift a portion of the gas stream 142 prior to introduction, for example, into a hydrogen purification apparatus for separation and recycle of hydrogen to methanol synthesis at 130B. In such embodiments, a portion of the gas stream 142 can be recycled to methanol synthesis at 130B (e.g., via the hydrogen purification apparatus) without shifting. Similarly, at least a portion of a shifted stream from an upstream shift reactor (e.g., a portion of shifted syngas stream 133 from water gas shifting at 130A) can be introduced into a hydrogen purification apparatus prior to introduction into methanol synthesis at 130B. In embodiments, a portion of the syngas can be diverted for the isolation of a purified hydrogen product.

As noted above, a methanol synthesis section 30/130 of this disclosure can comprise syngas compression C2. For example, following water removal via cooling B3, the dry syngas is compressed to provide a compressed stream 136 prior to methanol synthesis at 130B. As noted herein, compression of the dry syngas can be effected, in embodiments, without the use of a gas and/or steam (wherein the steam is produced via combustion of hydrocarbon(s)) turbine, in embodiments. For example, in embodiments, such as for example a retrofit application, an electrically-driven compressor or electric motor or a steam turbine operated with electrically-produced steam may be utilized to compress the dry syngas prior to methanol synthesis at 130B. As noted above, the methanol synthesis section 30/130 can comprise syngas compression C2. For example, the dry gas stream 134 may be introduced to the methanol synthesis reaction 130B via compression C2 with one or more compressors. Compression C2 can raise the pressure to about 1000 to about 3000 psia in compressed syngas stream 136, in embodiments.

As noted above, a methanol synthesis section 30/130 of this disclosure can comprise synthesis gas heating A4. Syngas heating may be utilized to heat the dry, compressed syngas stream 136 to a desired methanol synthesis reaction temperature in heated syngas feed 137. In embodiments, the desired methanol synthesis reaction temperature can be in a range of from about 150°C to 260°C, depending on the design of the methanol synthesis reactor.

As per this disclosure, a majority, greater than 20, 30, 40, 50, 60, 70, 80, or 90%, or substantially all of the heating of the compressed stream 135 via syngas heating A4 (and heat input indicated at Q9) may be effected electrically and/or otherwise does not require fuels combustion (e.g. via heat exchange with another process gas stream, such as, without limitation, cooled, shifted syngas stream 133), in embodiments.

As noted above, a methanol synthesis section 30/130 of this disclosure comprises methanol synthesis 130B. Thus, a methanol synthesis plant of this disclosure comprises methanol synthesis apparatus for carrying out the methanol synthesis 130B. The methanol synthesis section 30/130 can comprise one or more methanol synthesis reactors or catalyst beds for carrying out the methanol synthesis 130B. The number and placement of methanol synthesis reactors is generally known to those skilled in the art. For example a methanol synthesis reactor may be 1-pass methanol synthesis reactor, downstream of syngas compression and with a second compressor for recycle after crude methanol recovery, a 1-pass methanol synthesis reactor followed by a methanol synthesis reactor with recycle, 2 methanol synthesis reactors in series within the syngas recycle loop, or any of a variety of other configurations for methanol synthesis known to those of skill in the art.

The methanol synthesis involves the following three equations, Eqs. (7)-(9):

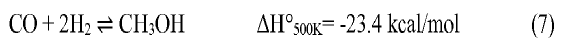

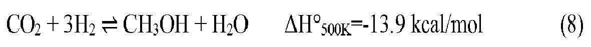

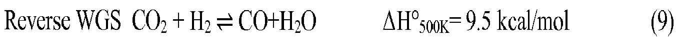

In the methanol synthesis, CO, CO₂, and H₂ in the syngas feed in stream 137 react to produce methanol via Equations (7) and (8). The reaction may be carried out, for example, at about 235 °C over a copper or zirconium containing catalyst. In embodiments, a mixture of copper and zinc oxides, supported on alumina, is utilized as catalyst. The reaction is exothermic (with heat removal indicated at Q10) and conversion is equilibrium limited. Heat removal can be integrated into the reactor design, for example by interstage cooling between multiple reactors and/or catalyst beds within the same reactor, through water cooling, or through the injection of cold gas including syngas. The water-gas shift reaction (Eq. (8)) also takes place over the catalyst. As the Equations (7), (8), and (9) are reversible, it is important to control process conditions regarding temperature, pressure, and synthesis gas mixture. Many reactor designs have been implemented, and the main reactor design differences are in how the reaction temperature is controlled. As per this disclosure, any suitable methanol synthesis technology may be utilized, as long as the operation follows the energy constraints provided herein. For example, in embodiments, a methanol synthesis reactor is operated with cooling to maintain a desired methanol synthesis temperature effected electrically. Electrically controlling the reactor temperature, as per this disclosure, may enable improved temperature control, in embodiments. For example, in embodiments a combination of heating and cooling techniques may be used for the methanol synthesis in methanol synthesis section 30/130. In embodiments, an electric heater is used to heat the syngas to the methanol synthesis reaction temperature. In embodiments, a vapor compression heat pump is used to heat the syngas to the methanol synthesis reaction temperature. In embodiments, a heat pump (vapor compression, chemical, absorption, or adsorption) is used to cool the methanol synthesis reactor. In embodiments a thermoelectric device is used to heat, cool, or simultaneously heat part of the reaction and cool another part of the reaction. In embodiments the heat of the methanol synthesis reaction is removed by heating water to make steam, by heating another heat transfer fluid, and/or by heat exchange with a process gas or gaseous heat transfer media. For example, in embodiments, heat produced at Q10 and/or Q11 may be utilized to provide some or all of the heat input needed at Q9 via heat exchange between methanol product stream 135 and compressed syngas stream 136. The product methanol may be removed from methanol synthesis 130B via methanol product stream 135.

According to embodiments of this disclosure, methanol synthesis 30/130 can be effected with a reduced usage of non-carbon based energy, the use of renewable energy, and/or the use of electricity (e.g., electricity from renewable and/or non-renewable source(s)). For example: in embodiments, the heat removal Q6 required to attain a desired water gas shifting temperature by cooling B1 can be electrically provided; the heat removal Q7 required to effect syngas cooling B2 can be electrically provided; the heat removal Q8 for cooling and water removal/condensing B3 can be electrically provided; the compression provided at syngas compression C2 can be effected via a motor or an otherwise electricity-driven compressor rather than via a steam or gas-driven turbine or via a turbine driven by electrically-produced steam; the heating Q9 needed to reach a desired methanol synthesis temperature at heating A4 can be electrically provided; electrical cooling may be utilized for heat removal Q10 from methanol synthesis reactor(s) of methanol synthesis 130B, or a combination thereof.

As noted above, a methanol synthesis plant of this disclosure can comprise a methanol purification section 40/140. The methanol purification section 40/140 can be operable to remove a crude methanol product from the methanol synthesis product, remove one or more components (e.g., light ends, heavy byproducts, water, or a combination thereof) from the crude methanol product, recycle syngas vapor to methanol synthesis 130B, and/or purge gas from the system. In embodiments, any suitable methanol purification system known in the art can be utilized, so long as it has been modified to include non-carbon based energy, renewable energy, and/or electricity (e.g., has been electrified to use renewable and/or non-renewable electricity for power) as per this disclosure.

In embodiments, the methanol purification section 40/140 can comprise crude methanol separation apparatus, a recycle compressor to recycle syngas vapor to the methanol synthesis apparatus, a purge gas system operable to purge gas (*e.g*., a portion of the unconverted synthesis gas enriched in inerts and other less desired components, such as CO₂ and N₂) from the methanol purification section, a light ends removal apparatus, a heavy byproducts separation apparatus, a methanol/water separation apparatus, associated heating, cooling, and/or compressing apparatus, or a combination thereof, as described further hereinbelow.

As noted above, a methanol purification section 40/140 can comprise cooling. For example, cooling B4 may be utilized downstream of methanol synthesis 130B (with heat removal indicated at Q11). In embodiments, the methanol synthesis reactor effluent is cooled at cooling B4 to near ambient temperature, where the crude methanol and formed water from the reaction condenses. In embodiments, a majority, greater than 20, 30, 40, 50, 60, 70, 80, or 90%, or substantially all of the energy consumed to provide the heat removal Q11 needed at syngas cooling B4 is provided from a non-carbon based energy source, from a renewable energy source, such as renewable electricity, and/or from electricity (from a renewable and/or non-renewable source). In embodiments, some or a majority of a number of pumps utilized to circulate cooling water are electrically driven. Desirably, as noted hereinabove, heat removal Q11 is effected via heat exchange with compressed syngas stream 136 to provide the heat input Q9 at syngas heating A4.

As noted above, a methanol purification section 40/140 can comprise crude methanol recovery at 140A. In embodiments, crude methanol recovery is effected by a gas/liquid separator operable to separate the water and methanol condensed at cooling B4 from the gas (e.g., syngas vapor).

As noted above, the methanol purification section 40/140 can comprise compression apparatus (also referred to herein as a recycle compressor) C3 operable to recycle unconverted syngas to the one or more methanol synthesis reactor(s). The unreacted gas may be compressed via recycle compressor C3, and recycled back to methanol synthesis 130B via gas recycle stream 144. In embodiments, recycle compression may also be combined with syngas compression. The production of synthesis gas from methane via steam reforming produces three moles of hydrogen for every mole of carbon monoxide (Eq. (3)), while the methanol synthesis (Eq. (6)) consumes only two moles of hydrogen gas per mole of carbon monoxide. To deal with excess hydrogen, carbon dioxide may be injected into the methanol synthesis reactor(s), in embodiments. Thus, in embodiments, carbon dioxide may be introduced into the one or more methanol synthesis reactor(s) via compressor C2 or C3.

Residual methane and other inerts, such as nitrogen and argon, as well as unreacted CO, H₂ and CO₂, build up in the recycle gas stream. These can be purged from the recycle gas via a purge gas system. As noted above, the methanol purification section 40/140 can thus comprise a purge gas system operable to purge gas (e.g., including methane, nitrogen, argon) from the methanol purification section 40/140. In embodiments, to handle residual gas (e.g., methane, nitrogen, argon) build-up in the recycle gas in gas recycle stream 144, a portion of the gas is purged from the recycle gas via a purge gas system 140B. Purge gas in line 143 may enter purge gas system 140B, and is removed from the methanol synthesis plant via purge line 105'. In embodiments, a purge stream 105" is utilized to extract purge gas upstream of recycle compressor C3. In embodiments, all or a part of the purge gas may be introduced into pretreating section 10/110 or syngas synthesis section 20/120 as a component of the reformer feed. Purge gas system 140B can involve heat removal, which can be provided electrically, in embodiments. Additional steps, such as water scrubbing, can be used to help improve the per pass recovery of methanol and avoid losses. Some purge gas systems include additional separations to recover specific purge gas components, such as hydrogen. Such additional steps, separations, and apparatus therefor (e.g., hydrogen removal and/or hydrogen purification apparatus, water scrubbing and/or water scrubbing apparatus) are within the scope of this disclosure. In embodiments, fractionation, adsorption, gas permeable membrane separation, molecular sieves, absorption may be used as separation methods. In embodiments, chemical reactions (e.g., methanation) may be used to enhance or enable the practical feasibility or efficacy of the separation methods. In embodiments, such additional separations are also electrified and/or otherwise effected at least in part without the use of non-renewable carbon-based energy. In embodiments, purge gas is not burned for its fuel value, as is common in conventional operation. In embodiments, less than or equal to about 50, 40, 30, 20, 10, 5, 4, 3, 2, or 1 weight percent (wt%) of the purge gas is combusted (e.g., within the syngas synthesis section 20/120 or elsewhere in the methanol synthesis plant). In embodiments, purge gas is not burned for its fuel value, but is separated into one or more different streams comprising specific compounds or groups of compounds, where such one or more different streams are further processed in specific beneficial manners. For example in embodiments, methane and/or nitrogen are removed (e.g., via fractionation) from the purge gas. In embodiments, the methane and nitrogen are removed from the purge gas by cryogenic fractionation that is cooled by a vapor compression heat pump where the compressor is powered by an electric motor. In embodiments, the methane is recycled for use as a feed for methanol synthesis. In embodiments, nitrogen and/or methane removed from the process is sent for use in an ammonia synthesis process. In embodiments, inert gases such as nitrogen and argon are removed from the process while some or all of the remaining components (e.g., CO, CO₂, methane, and/or hydrogen) are recycled. In embodiments, hydrogen is separated from the purge gas in purge gas stream 105' or 105", and the separated hydrogen is recycled directly to the methanol synthesis section 30/130 and/or utilized for some other duty within the plant (e.g., utilized in the feed pretreatment section, such as for hydrodesulfurization via Equation (1) or burned to provide heat). In embodiments, hydrogen separated from the purge gas in purge gas stream 105' or 105" is removed from the process and consumed elsewhere.

In embodiments, the purge gas separation system comprises multiple systems, taking gas streams from different parts of the plant, for example, a high pressure purge gas system for the methanol synthesis loop purge gases and a low pressure purge gas system for the crude methanol degassing column light ends. In embodiments, different streams of the multiple systems can be connected together in series or parallel in the overall purge gas processing scheme. In embodiments a compressor is used to increase the pressure of a low pressure purge gas to enable recycle or treatment in a purge gas separation system operating at higher pressure. In embodiments, the purge gas system is a single, unified processing unit.

In embodiments, the methanol synthesis plant further comprises a hydrogen separation apparatus downstream from the methanol synthesis section and configured to separate hydrogen produced in the reformer from a purge gas, thus providing a separated hydrogen stream, a hydrogen purification apparatus configured to purify the separated hydrogen stream, thus providing a purified hydrogen stream; and/or one or more fuel cells operable to make electricity from the purified hydrogen stream. The methanol synthesis plant can further comprise storage apparatus configured for storage of the separated hydrogen, the purified hydrogen, or a combination thereof, such that the separated hydrogen, the purified hydrogen, or the combination thereof can be stored when electricity is readily available, and whereby the stored hydrogen can be utilized to make electricity in the one or more fuel cells when other sources of electricity are not readily available and/or are not available at a desirable price. In embodiments, the separated hydrogen is sent to an ammonia synthesis and/or other process.

As noted above, a methanol purification section 40/140 can comprise separating methanol from the crude methanol product in crude methanol stream 146. Any suitable separations can be utilized, so long as they are effected in accordance with the utilization by the methanol synthesis plant of non-carbon based energy, renewable energy, and/or electricity (e.g., renewable and/or non-renewable electricity) as per this disclosure. The temperature in the purification section for separating the methanol from the crude methanol stream is generally 150°C or lower, in embodiments. In embodiments, separating methanol from the crude methanol stream comprises removing light ends, heavy byproducts, and/or water from the methanol to provide a purified methanol product 145.

In embodiments, separating methanol from the crude methanol stream 146 comprises removing light ends from the crude methanol product at 140C. In embodiments, a degassing column is utilized at 140C to separate the crude methanol in stream 147 from light gases. Heat (as indicated at Q12) may be introduced to effect this light ends removal, in embodiments. As per this disclosure, in embodiments, a majority, greater than 20, 30, 40, 50, 60, 70, 80, or 90%, or substantially all of the net heat input (Q12) needed at product light ends removal 140C is provided from a non-carbon based energy source, from a renewable energy source, such as renewable electricity, and/or from electricity (e.g., electricity from a renewable and/or a non-renewable source).

In embodiments, separating methanol from the crude methanol stream further comprises separating heavy byproducts at heavy byproducts separation 140D. Hydrocarbon and higher alcohol byproducts that are formed in the methanol synthesis reaction at methanol synthesis 130B can be separated by fractionation. These heavy byproducts can be taken out either as a side draw from either the light ends removal apparatus (e.g., a degassing column) utilized at light ends removal 140B or from a methanol/water separation apparatus (e.g., a methanol purification tower) at methanol/water separation 140E, or by a top or bottom product from a separate distillation tower. For example, a methanol stream 147 from light ends removal 140C can be introduced into a heavy byproducts separation apparatus at 140D, wherein heavy byproducts can be removed from the methanol stream 148. Heat (as indicated at Q13) may be introduced or removed to effect this heavy byproducts removal, in embodiments. As per this disclosure, in embodiments, a majority, greater than 20, 30, 40, 50, 60, 70, 80, or 90%, or substantially all of the net heat input or removal (Q13) needed at heavy byproducts separation 140D is provided from a non-carbon based energy source, from a renewable energy source (e.g., from a non-fossil fuel based energy source E_{NF}), such as renewable electricity, and/or from electricity (e.g., electricity from a renewable and/or non-renewable source).

In embodiments, separating methanol from the crude methanol stream further comprises separating methanol and water at methanol/water separation 140E. In embodiments, methanol and water are distilled to purify methanol, and thus provide a purified methanol product 145 and water 149. Where sufficiently free from detrimental contaminants or trace chemistry modifiers, such as NaOH, water 149 can be recycled to a shift reactor(s) for shifting at 130A and/or to steam generation 112. Alternatively, water 149 can be cleaned prior to recycle.

The methanol/water separation at 140E can be effected in a single fractionating tower (e.g., distillation column) or multiple towers (e.g., multiple distillation columns) for heat integration purposes. Heat (as indicated at Q14) may be introduced or removed to effect this methanol/water separation, in embodiments. As per this disclosure, in embodiments, a majority, greater than 20, 30, 40, 50, 60, 70, 80, or 90%, or substantially all of the net heat input or removal (Q14) needed at methanol/water separation 140E is provided from a non-carbon based energy source E_{NC}, from a renewable energy source (e.g., from a non-fossil fuel based energy source E_{NF}), such as renewable electricity, and/or from electricity (e.g., electricity from a renewable and/or non-renewable source). In embodiments, the temperatures of individual trays on a distillation column of methanol/water separation 140E are controlled electrically to improve separation efficiency.

According to embodiments of this disclosure, methanol purification can be effected with a reduced usage of non-carbon based energy and/or non-renewable energy, with the (increased) use of renewable energy, and/or with the use of electricity (e.g., renewable electricity from renewable and/or non-renewable source(s)). For example, in embodiments, the net heat removal Q11 (e.g., any heat removal needed beyond that provided by heat exchange with another process stream) needed at syngas cooling B4 can be electrically provided; the compression provided at recycle compressor C3 can be effected via a motor or an otherwise electricity-driven compressor rather than via a steam or gas-driven turbine or via a turbine driven by electrically-produced steam; the net heat input or removal Q12 needed for light ends removal at 140C can be electrically provided; the net heat input or removal Q13 needed for heavy byproduct separation 140D can be electrically provided; the net heat input or removal Q14 needed for water/methanol separation 140E can be electrically provided; or a combination thereof.

As noted in Table 1 hereinabove, electrical heating provides a higher energy efficiency than heating via fuels combustion. In embodiments, distillation column feeds can be heated by mechanical heating, resistive heating elements, impedance, thermoelectric devices, and/or electromagnetic waves heating the process fluid or another media from which the heat is transferred. In embodiments, distillation columns can be heated electrically at the reboilers and/or in individual sections by mechanical heating, electric resistance heaters, impedance, thermoelectric devices, and/or electromagnetic waves heating the process fluid or another media from which the heat is transferred. Heat pumps (vapor compression, absorption, adsorption, or chemical) may be used. Thermoelectric devices may be used as heat pumps, in embodiments. In embodiments, distillation column condensers and product coolers can be cooled by electrically powered air coolers, cooling water circuits, thermoelectric devices, and/or heat pumps.

In embodiments, one or more of the Q1-Q15 can be provided by recovered heat, rather than by externally supplied energy. In embodiments, a majority, greater than 20, 30, 40, 50, 60, 70, 80, or 90%, or substantially all of the net heat input or removal needed within the methanol synthesis plant, the feed pretreating section, the syngas synthesis section, the methanol synthesis section, the methanol purification section, or a combination thereof is provided from a non-carbon based energy source E_{NC}, from a renewable energy source (e.g., from a non-fossil fuel based energy source E_{NF}), such as renewable electricity, and/or from electricity (e.g., electricity from renewable and/or non-renewable source(s)). In embodiments, a majority, greater than 20, 30, 40, 50, 60, 70, 80, or 90%, or substantially all of the net heat input or removal needed within the methanol synthesis plant, the feed pretreating section, the syngas synthesis section, the methanol synthesis section, the methanol purification section, or a combination thereof is provided from a non-carbon based energy source E_{NC}, from a renewable energy source (e.g., from a non-fossil fuel based energy source E_{NF}), such as renewable electricity, and/or from electricity (e.g., electricity from renewable and/or non-renewable source(s)).

In embodiments, a majority, greater than 20, 30, 40, 50, 60, 70, 80, or 90%, or substantially all of the energy needed for compression (e.g., at feed gas pressure adjustment C1, syngas compression C2, and/or recycle compressor C3) within the methanol synthesis plant, the feed pretreating section, the syngas synthesis section, the methanol synthesis section, the methanol purification section, or a combination thereof is provided from a non-carbon based energy source, from a renewable energy source, such as renewable electricity, and/or from electricity (e.g., electricity from renewable and/or non-renewable source(s)). For example, an electric motor, an electrically-driven turbine, and/or a turbine driven by steam produced electrically may be utilized to provide compression throughout the methanol synthesis plant or one or more sections thereof. In embodiments, a majority, greater than 20, 30, 40, 50, 60, 70, 80, or 90%, or substantially all of the compressors are replaced by or utilize an electric motor, an electrically-driven turbine, and/or a turbine driven by steam produced electrically.

In embodiments, electricity can be used to provide the motive force for fluids. For example, electricity can be used to power pumps to move and/or pressurize liquids, and/or to power air blowers and/or fans. In embodiments, a fraction, a majority, or all (e.g., 20, 30, 40, 50, 60, 70, 80, 90, or 100%) of the number of pumps utilized in the methanol synthesis plant are electrified.

In embodiments, electricity is utilized to produce slightly colder (e.g., 2, 5, 10 or 15°C colder) cooling water than conventional.

As noted above, when utilizing electricity from a renewable source that has a potentially or known intermittent supply (e.g., an intermittent energy source or IES), various steps can be taken to maintain operation of the methanol synthesis plant, according to embodiments of this disclosure. Such handling of an IES can be as described in U.S. Provisional Patent Application Nos.__________ and __________, entitled *Use of Intermittent Energy in the Production of Chemicals,* which are being filed concurrently herewith, the disclosure of each of which is hereby incorporated herein for purposes not contrary to this disclosure. For example, in embodiments, compressed hydrogen is stored for intermittency of electric supply. Alternatively or additionally, one or more cryogenic liquids can be stored for intermittency of electric supply. Alternatively or additionally, heat can be stored for intermittency of electric supply. Alternatively or additionally, batteries can be kept for intermittency of electric supply. Backup power for key components may be provided; non-renewable electricity may be utilized as a back-up for intermittent renewable electricity, in embodiments. For example, such backup power may be produced via apparatus driven by compressed gas or a flywheel. Alternatively or additionally, in embodiments, a feed (e.g., a natural gas feed) can be compressed and stored when electricity is available and expanded through a power-generating turbine to provide feed and electricity during times of low electric supply. Alternatively or additionally, recovered H₂ (e.g., from purge gas 143, 105', 105") can be pressurized and stored when electricity is available and used to generate electricity using a fuel cell during times of low electric supply.

Electrification of the methanol synthesis plant of this disclosure can be provided via an electricity supply that can be high voltage or low voltage. The electric devices can be operable or operated on alternating (single or multiphase) or direct current.

In embodiments, steam generated by the combustion of fuels or produced solely for heat and/or energy transfer is not utilized in a methanol synthesis plant and method of this disclosure (e.g., in the pretreating section 10, the syngas synthesis/reforming section 20/120, the methanol synthesis section 30/130, and/or the methanol purification section 40/140). In this manner, a methanol synthesis plant according to this disclosure can be operated, in embodiments, without an elaborate steam heat and/or energy transfer system (which may be conventionally utilized in a chemical plant for producing methanol). In some applications, for example where steam is utilized within a reactor as a feed component and/or diluent, such steam may be produced via heat transfer with a process stream within the methanol synthesis plant and/or may be produced electrically. In embodiments, steam generated via heat transfer with a process stream may be superheated using electricity. In embodiments, steam is not utilized throughout the methanol synthesis plant as a commodity or utility. In embodiments, a methanol synthesis plant of this disclosure is essentially steam-free, or utilizes substantially less steam (e.g., uses at least 10, 20, 30, 40, 50, 60, 70, 80, 90, or 100 volume percent (vol%) less steam) than a conventional plant for producing methanol. For example, a conventional plant for producing methanol may utilize steam production for reboilers of distillation columns of the feed pretreating section 10 and/or the methanol purification section 40/140, may utilize steam production to drive steam turbines for compressing process and/or recycle streams, or may utilize steam production to drive steam turbines for refrigeration. In embodiments, steam is not produced for these operations in a methanol synthesis plant according to this disclosure, or substantially less steam is produced (e.g., at least 10, 20, 30, 40, 50, 60, 70, 80, 90, or 100 volume percent (vol%) less steam). In embodiments, steam is used as a heat transfer fluid, but is not used to do mechanical work (e.g., to drive a compressor or pump.) In embodiments, the steam generated for these operations is primarily (e.g., of the total steam utilized, the greatest percentage is electrically produced), mainly (e.g., greater than 50% of the steam is electrically produced) or substantially all electrically produced. In embodiments, the steam utilized as a reactant or diluent is primarily (e.g., of the total steam utilized, the greatest percentage is electrically produced), mainly (e.g., greater than 50% of the steam is electrically produced) or substantially all electrically produced.

In embodiments, in a methanol synthesis plant or process of this disclosure, more energy is utilized directly 'as-is', for example, utilizing heat from a hot product effluent stream to heat a feed stream, rather than being transformed, e.g., via the generation of steam and the conversion of the thermal energy to mechanical energy via a steam turbine. According to embodiments of this disclosure, the use of energy directly can increase the energy efficiency of the methanol synthesis plant, for example by reducing energy efficiency losses that occur when heat is converted to mechanical energy.

As energy consumption is a large fraction of the operating costs of a traditional methanol synthesis plant, increasing energy efficiency (e.g., via electrification) as per this disclosure and/or utilizing methane conventionally burned to provide heat for reforming and/or burned for compression (e.g., burned to produce steam for a steam turbine or burned for a gas turbine) to produce additional methanol may provide economic advantages over a conventional methanol synthesis plant. Concomitantly, the reduction of the burning of fossil fuels (e.g., natural gas, methane) as a fuel enabled via this disclosure provides for reduced greenhouse gas (GHG) emissions relative to a conventional methanol synthesis plant in which hydrocarbons are burned as fuel. In embodiments, GHG emissions (e.g., carbon dioxide emissions) are reduced by at least 5, 10, 20, 30, 40, 50, 60, 70, 80, 90, or 100% relative to a conventional methanol synthesis plant in which hydrocarbons are burned as fuel. In embodiments of this disclosure, the amount of CO₂ produced per ton of methanol produced is reduced to 0.30, 0.25, 0.2, 0.1, 0.05, or 0 tons CO₂ per ton methanol. In embodiments, energy efficiency (e.g., reduced energy losses) is increased by the elimination of the flue gas, since the loss of heat contained in the flue gas to the atmosphere is eliminated. In embodiments, energy efficiency (e.g., reduced energy losses) is increased by a decrease in or elimination of the use of steam to do mechanical work. In embodiments, the energy efficiency of the process is increased such that the specific energy consumption (the total net energy input, including feed methane, fuel, and electricity, and giving credit for byproducts produced, to the process divided by the production rate) is 34, 33, 32, 31, 30, 29, 28, or 27 GJ/ton of methanol produced, where the specific energy consumption is calculated using the higher heating value of the fuel, feed, and byproducts. In embodiments, aspects of this disclosure can lead to an increase in carbon efficiency of a process, i.e. to a fraction of carbon consumed in the process that reappears as a useful product. In embodiments, the carbon efficiency of the process is greater than or equal to about 82, 84, 86, 88, 90, 92, 94, 95, 96, 97, 98, 99, or 100%.

Conventionally, the energy required for unit operations in chemical processes is generally provided by the burning of fossil fuels, especially natural gas. Herein-disclosed are systems and methods by which this energy input can be reduced or replaced, in embodiments, with non-carbon based energy, renewable energy, such as renewable electricity, and/or by electricity from any source (e.g., renewable and/or non-renewable), such that energy efficiency is improved. The herein-disclosed use of non-carbon based energy, renewable energy (e.g., non-fossil fuel based energy), and/or electricity in the production of chemicals, such as the production of methanol via syngas reforming or partial oxidation, increases energy efficiency of and/or decreases and/or eliminates carbon dioxide emissions from and fossil fuel consumption within the methanol synthesis process.

In embodiments, no steam is used as an energy transfer medium anywhere within the process. In embodiments, steam is only used as an energy transfer medium to move thermal energy within the process, e.g., to facilitate the use of heat recovered from cooling one process stream into the heating of a second process stream. In embodiments, some or all of the heat recovered from cooling one or more process streams is used only to heat one or more other process streams via direct heat exchange (i.e., via direct or indirect heat transfer with another process stream without utilizing steam as a heat transfer intermediate). In embodiments, no steam is used to do mechanical work within the methanol synthesis plant. In embodiments, no flue gas is produced via the combustion of a fuel, a carbon-based fuel, a fossil fuel, or a combination thereof within the methanol synthesis plant. In embodiments, hydrogen recovered from the methanol synthesis process (e.g. from purge gas stream 143, 105', 105") is sent to an ammonia synthesis plant. In embodiments, hydrogen recovered from the process (e.g. from purge gas stream 143, 105', 105") is sent to another chemical process, e.g., for use in the refining of oil and/or oil derivatives.

In embodiments, electric heating is used to impose a temperature profile on a reactor (e.g., a reformer) of syngas synthesis section 20/120, a methanol synthesis reactor and/or a water-gas shift reactor, such as at methanol synthesis 130B and water-gas shift 130A, of a methanol synthesis section 30/130, or a combination thereof. In embodiments, mechanical heating is used to heat a feed to a reactor (e.g., a reformer) of syngas synthesis section 20/120, a methanol synthesis reactor and/or a water-gas shift reactor, such as methanol synthesis 130B and water-gas shift 130A, of a methanol synthesis section 30/130, or a combination thereof.

### EXAMPLES

The embodiments having been generally described, the following examples are given as particular embodiments of the disclosure and to demonstrate the practice and advantages thereof. It is understood that the examples are given by way of illustration and are not intended to limit the specification or the claims in any manner. As utilized herein between components or process steps, a slash "/" indicates and/or, for example, 'feed preheating A1/A2/A3' indicates 'feed preheating A1, A2, A3, or a combination thereof'. Similarly, '140D/140E' indicates '140D, 140E, or both'. Although specific operating conditions are utilized in the process simulations of the Examples, it is to be understood that other operating conditions (e.g., temperatures, flow rates, etc.) may be apparent to those of skill in the art upon reading this disclosure and are intended to be included within the scope of this disclosure.

### Comparative Example 1

A process simulation was performed to determine the heat and mass flows for a typical process III for the production of methanol from natural gas. The process simulation utilized in this Comparative Example 1 was made using Aspen Plus®, a commercial chemical process simulation software tool. It does not represent a specific operating plant, but it is representative of a typical plant or process III as described hereinbelow; the design parameters were taken from knowledge of specific plants, as well as literature information on typical process operations. Although variations will be obvious to one skilled in the art, this Comparative Example 1 represents a typical process III that can be used as a basis for comparing the effects of electrification modifications according to embodiments of this disclosure.

Process III of Comparative Example 1 is configured to produce 5,000 metric tons per day of methanol. If operated for 8,000 hours in a year, this would result in the production of 1.67 million tons of methanol, although variations in downtime due to upsets and maintenance could increase or reduce this output. This size is typical of contemporary large methanol plants.

The natural gas composition used in Comparative Example 1 and all of the subsequent Examples and Comparative Examples is typical of natural gas found in some parts of the Middle East. It contains 88.9 mol% methane, 2.3 mol% ethane, 0.8 mol% C₃+ hydrocarbons, 7.8% N₂, and 0.2% CO₂. It has a higher heating value (HHV) of 48.0 GJ/t.

135 metric tons per hour (t/h) of natural gas feed 205 are fed to the feed pretreatment section where it underdoes pressure adjustment (e.g., at C1) and desulfurization (e.g., at 210A) (hydrogen addition not shown) after heating (e.g., at feed pretreatment A1) to the desired desulfurization temperature of 385°C. Water 214 and steam 213 are added to provide a reforming feed 215 (e.g., with a steam to carbon molar ratio of 1.8), and the feed 215 is preheated (e.g., at feed pretreatment A1/A2/A3) and fed to a steam methane reformer of 220A where the feed is passed over a reforming catalyst while being heated in the radiant section of a reforming furnace 220'. The effluent 221 of the steam reformer is passed to a partial oxidation reforming unit 220B where 95 t/h of oxygen 228 is added and reacted with the gas stream. The temperature of the effluent 225 exiting the partial oxidation reformer 220B is 976°C. The reforming product 225 is cooled down (e.g., in syngas cooling B1/B2) to close to the mixture dew point before further cooling of the cooled syngas 231 to 45°C in a water condensing and removal section (e.g., water condensing B3) to generate an essentially dry syngas stream 234 and a recycle water stream 238. The dried syngas 234 is passed to a syngas compressor C2 where the pressure is increased from 35 to 99 bar. The compressed, dried syngas 236 is mixed with recycled syngas 244 and preheated (e.g., at syngas heating A4) to 165°C. The preheated syngas 237 is fed to the methanol reactors of 230B where it is heated to the final reaction temperature and passed over a methanol synthesis catalyst. The methanol synthesis reaction, which is exothermic, is cooled (e.g., at cooling B4, which is integrated in this example into the methanol synthesis reactors of 230B) by water which is vaporized, sent to the water addition section 216 and condensed to provide the heat required for the water addition to the feed. The methanol reactor effluent 241 is cooled down to 45°C in crude methanol recovery/separation 240A where vapor and liquid phases are separated. The vapor phase 242 is passed to the recycle compressor C3 after taking 34 t/h off as a purge gas stream 205'. This purge gas 205' from the synthesis loop is combined with the low pressure off gases 247' from a degassing column of light ends removal 240C to provide a 44.6 t/h stream containing 53 mol% H₂, 19 mol% N₂, 13 mol% CH₄, 10 mol% CO₂, and 4% other compounds; this stream is then burned in the reformer furnace 220' for fuel, along with 10.3 t/h of natural gas 205". The liquid stream 247 is fed to a degassing column at 240C where it is heated to remove light gases 247' dissolved in the crude methanol stream 246. The degassed crude methanol 247 is fractionated to provide a substantially pure methanol product stream. This is effected, in this Comparative Example 1, via a 2 column sequence where a partial recovery 245A of the methanol is taken in the first column 240D and the remaining methanol 245B is purified by fractionation in the second column 240E. Heavy byproducts are purged via a small side stream in the column (not shown). The overhead streams (245A/245B) of each of the columns (240D/240E, respectively) are cooled in methanol product coolers at B5 to 45°C before being sent to methanol product handling and storage as methanol product 245C. The water 249A separated from the methanol is cooled as well, e.g., in separated water cooling at B6, before discharging as cooled water 249B. Water 238 that was condensed from the syngas 231 is reheated (e.g., at process water heating A5) and recycled to the water addition 216. A small fraction of the water can be purged from the system to prevent the buildup of impurities.

The major energy consumers of Comparative Example 1 are (see Table 2, discussed further hereinbelow): (1) heat (e.g., Q4) supplied to the steam reformer, (2) generation of the steam required as feed for syngas production, (3) methanol distillation (e.g., in a first methanol separation column of 240D and a second methanol separation column of 240E), (4) reformer feed preheat (e.g., at feed preheating A1/A2/A3), (5) methanol reactor feed preheat (e.g., at syngas heating A4), (6) energy (e.g., for work W2) to drive the syngas compressor C2, and (7) energy to drive an air separation unit ASU for the production of oxygen 228. Smaller amounts of energy are used for a variety of other purposes. As is common practice, very little electricity is consumed in Comparative Example 1, primarily for some smaller pumps; the electrical demand for process users is only 1.7 MW. A significant amount of the energy used can be obtained by heat exchange with the product streams as they are cooled, most notably the hot syngas (e.g., 221/225) from the reformer (e.g., at 220A/220B) and the product from the methanol reactor(s) at 230B/B4. The remaining energy is supplied as combusted fuels in the reforming furnace 220' and a steam boiler 260. The reforming furnace 220' serves multiple purposes, supplying heat for the steam reforming reaction from the radiant section, providing some feed preheating in the convection section, and supplying heat in the convection section for use in high pressure steam 262A generation. Cooling of the syngas is also utilized in the generation of high pressure steam 262A. The remaining high pressure steam 262B is generated by the steam boiler 260. The high pressure steam 262A/262B is directed through a system of steam turbines (e.g., 363/265) to drive the large power users of the plant. In Comparative Example 1 the syngas charge compressor C2, the air separation unit ASU, and the cooling water pumps are partly extracting plus condensing turbines (e.g., indicated at 263). The gas feed booster compressor, reformer air/flue gas fans, and the syngas recycle compressor C3 are backpressure turbines (e.g., indicated at 265) that exhaust 182 t/h low pressure steam 266 that is used for heating in the process III. In Comparative Example 1, there are two major locations which utilize external sources of energy. The first is in the reformer at 220A, which consumes 10.3 t/h of natural gas 205" with a contained chemical energy (high-heating value, or HHV) of 138 MW plus 44.6 t/h of process off gases comprising purge gas 205' from the synthesis loop and the low pressure off gases 247' having a contained chemical energy (high-heating value, or HHV) of 252 MW. The remaining external energy is supplied by a steam boiler 260 that converts boiler feed water BFW 261A to high pressure (HP) steam 262B utilizing 9.7 t/h of natural gas fuel 261B with a contained chemical energy of 130 MW. How to most efficiently allocate this energy to the various consumers of energy in the process with the highest efficiency is an engineering problem that can be addressed by one of skill in the art upon reading this disclosure via careful matching of temperatures, types of energy, and energy content. Some of the energy can be transferred directly via heat exchange, while some can be converted to steam that can either be used for heat exchange or to do mechanical work, such as drive a compressor. In Comparative Example 1 a typical strategy has been adopted for matching heat inputs and outputs, but other arrangements are possible, as will be obvious to one skilled in the art upon reading this disclosure. The use of combustion furnaces to supply the external energy input needed for the process comes with a concomitant disadvantage - the stack or flue gas from these furnaces contains energy that cannot be usefully recovered because of its low temperature. For example, in the process of Comparative Example 1, this unrecovered energy, sometimes referred to as stack losses, amounts to 71 MW at the reformer furnace 220' of steam reforming 220A and 26 MW at the steam boiler 260. Energy is also lost in several process steps, primarily in water condensing from various points such as from the syngas (e.g., at water condensing B3) because the temperature is too low for returning the energy to useful purposes.

Comparative Example 1 shows the energy integration configuration chosen for the purposes of this example. One with skill in the art would recognize a number of ways the heat recovery of the syngas cooling and reformer stack could be configured for raising the high pressure steam. For example, the ratios of how much energy is supplied in water preheating, vaporization, and steam superheating can be reassigned among the units according to the design and equipment configuration. Likewise the steam heat raised in the methanol synthesis reactor at 230B could be used for distillation heating (e.g., at 240D/240E) rather than reformer water (e.g., at A5) and steam heating (e.g., at 212). These and other heat integration choices are known art and for various reasons different choices could be taken by one skilled in methanol plant design, while remaining within the scope of this disclosure.

Table 2 shows the energy balance for the process III of Comparative Example 1. As seen in Table 2, an amount of 520 MW of chemical energy is supplied through the combustion of purge gas 205', process off-gas 247', and natural gas 205" in the reformer furnace 220' of steam reforming at 220A and of natural gas fuel 261B in the steam boiler 260; this represents all but 2 MW of the total energy input to the process III that is supplied from external sources. Including the heat supplied by the reforming furnace 220', a large amount of heat (1410 MW) is available within the process III. In addition to the methanol synthesis chemical reaction and cooling hot process streams, heat is internally generated within the process through the reaction of the natural gas with oxygen 228 in the partial oxidation reformer at 220B. A total of 705 MW is recovered from process sources; however, because of the available heat supplied by the steam turbines (e.g., 263/264), as well as the temperature and enthalpy requirements of the process, 385 MW of heat is rejected to cooling water. Additionally the steam system SS rejects 213 MW of heat to cooling water in the condensing turbines (e.g., 269). Furnace stack losses total 97 MW. Overall 705 MW of energy, 50% of the total heat available, is rejected rather than being taken as useful for the process.

Table 3 provides relevant energy use statistics for process III of Comparative Example 1. As seen in the data in Table 3, the carbon efficiency of process III, defined by the carbon contained in the product methanol (e.g., at 245C) divided by the carbon of the total natural gas consumed, is 76.8%. The combustion of this fuel results in the atmospheric emissions of 86 t/h of CO₂, or 0.69 million tons of CO₂ annually. Net specific energy consumption is 35.8 GJ (33.9 MMBTU) per ton of methanol produced. Net specific energy consumption includes the energy content of the natural gas feedstock (in higher heating value) for making methanol plus the external energy supplied in other forms such as electricity and natural gas for fuel; because the purge gas combusted in reforming furnace 220' is internal to the plant, the energy content of the natural gas feedstock includes the heat content of the purge gas.

### Example 1

Example 1 is a partial electrification process IV comprising partial electrification as per an embodiment of this disclosure of the methanol process III described in Comparative Example 1. In Example 1, the external steam boiler is removed and the energy supplied to that boiler by the combustion of natural gas is replaced by a smaller amount of renewable electrical energy that is used to power the air separation unit (ASU).

As in Comparative Example 1, an amount of 135 t/h of natural gas 205 is fed to process IV. The main difference in Example 1 is that the energy (e.g., W4) for the oxygen production unit ASU is not obtained from the steam system but is replaced with the power of an electric motor supplied by renewable energy. This reduces the total demand of high pressure steam such that running a steam boiler is no longer necessary and 9.7 t/h of fuel can be saved. There is additionally a small amount of excess high pressure steam 268A that is run through a turbine connected to an electric generator 263A to recover 2.4 MW of power.

Table 2 shows the energy balance for the partial electrification process IV of Example 1. An amount of 390 MW of chemical energy is supplied through the combustion of process gases (e.g., purge gas 205' and light gases 247') and natural gas (e.g., 205") and 24 MW of renewable electricity are supplied; this represents a total net energy input to process IV of 414 MW, or 21% less than in process III of Comparative Example 1. Total losses from the system from the flue gas, inefficiencies in the use of steam and electricity, and losses in process IV are 15% less than in process III of Comparative Example 1.

Table 3 provides relevant energy use statistics for process IV of this Example 1, according to an embodiment of this disclosure. The carbon efficiency of process IV is 82.0%, an improvement of approximately 5% over that of process III of Comparative Example 1. In this Example 1, the natural gas consumption results in the atmospheric emissions of 63 t/h of CO₂, or 0.54 million tons of CO₂ annually; this represents a 27% decrease over process III of Comparative Example 1. The 78,000 tons per year of natural gas saved via process IV can be used elsewhere, for example as a feed for an ammonia synthesis process and/or for another methanol plant. Net specific energy consumption of process IV is 33.9 GJ (32.2 MMBTU) per ton of methanol produced, which is 5% less than in that of process III of Comparative Example 1.

**Table 2**

| | Example (Process) | CE 1 (III) | 1 (IV) |
|---|---|---|---|
| CE 1 drawing label | | (values in MW) | (values in MW) |
| | Energy inputs to the battery limits | | |
| | Fired fuels | | |
| 25 | Process gases | 252 | 252 |
| 28 | Reformer NG | 138 | 138 |
| 29 | Boiler NG | 130 | 0 |
| | Electricity | 2 | 24 |
| | Total external energy inputs | 522 | 414 |

| | Process Heat sinks | | |
|---|---|---|---|
| | Heat rejection to cooling water | 385 | 385 |
| | High pressure steam generation | 331 | 331 |
| Q4 | Steam Reformer | 164 | 164 |
| Q3 | Saturator | 126 | 126 |
| Q16 | Second methanol column reboiler | 98 | 98 |
| Q14 | First methanol column reboiler | 89 | 89 |
| Q4 | Saturator to reformer feed heat | 80 | 80 |
| Q8 | Methanol reactor feed preheat | 52 | 52 |
| Q1 | Gas to desulfurization preheat | 28 | 28 |
| Q12 | Degassing column reboiler | 23 | 23 |
| Q2 | Medium pressure steam generation | 22 | 22 |
| Q20 | Oxygen preheating | 7 | 7 |
| Q7 | Process water preheating | 4 | 4 |
| | Methanation reactor preheat | | |
| | Drying gas heating | | |
| | Total process heat sinks | 1410 | 1410 |

| | Example (Process) | CE 1 (III) | 1 (IV) |
|---|---|---|---|
| | Process Heat sources | | |
| Q5 | Syngas cooling | 324 | 324 |
| | Reformer furnace (heat supplied) | 320 | 320 |
| Q10 | Methanol reactor effluent | 176 | 176 |
| Q9 | Methanol reactor cooling | 133 | 133 |
| Q22 | Low pressure steam use | 131 | 131 |
| Q17 | Second methanol column condenser | 110 | 110 |
| Q6 | Syngas condensing | 93 | 93 |
| Q15 | First methanol column condenser | 74 | 74 |
| Q11 | Syngas compressor intercoolers | 15 | 15 |
| Q13 | Degassing column condenser | 10 | 10 |
| Q21 | Desulfurization to sat gas cooling | 9 | 9 |
| Q19 | Methanol product cooler 1 | 7 | 7 |
| Q18 | Separated water cooling | 4 | 4 |
| Q19 | Methanol product cooler 2 | 2 | 2 |
| Q6 | Syngas process waste water cooling | 2 | 2 |
| | Low temp. electric heat supplied | 0 | 0 |
| | Medium temp electric heat supplied | | |
| | Off-gas furnace | | |
| | Methanated product cooling | | |
| | Methanation reaction cooling | | |
| | Refrigeration gas coolers | | |
| | Total process heat sources | 1410 | 1410 |

| | Power requirements | | |
|---|---|---|---|
| W2 | Syngas charge compressor | 30 | 30 |
| W4 | Oxygen production | 23 | 23 |
| W8 | Gas separation system | | |
| W6 | Cooling water circuit | 12 | 10 |
| W1 | NG feed booster compressor | 9 | 9 |
| W5 | Reformer air fans | 4 | 4 |
| W3 | Syngas recycle compressor | 3 | 3 |
| W7 | Other pumps, misc. | 2 | 2 |
| | Total power requirements | 83 | 81 |

| | Steam system | | |
|---|---|---|---|
| | Energy in the steam | 435 | 331 |
| | Work extracted | 81 | 59 |
| Q22 | Heat extracted | 131 | 131 |
| Q23 | Turbine condensate | 213 | 132 |
| | Other losses | 10 | 10 |
| | Total steam losses | 223 | 142 |

| | Losses | | |
|---|---|---|---|
| | Process | 385 | 385 |
| | Steam system | 223 | 142 |
| | Reformer stack | 71 | 71 |
| | Boiler/Furnace stack | 26 | 0 |
| | Electrical | 0 | 1 |
| | Total energy losses | 705 | 599 |

| Table 3 | | | | | | | | |
|---|---|---|---|---|---|---|---|---|
| Example (Process) | units | CE 1 (III) | 1 (IV) | 2 (V) | 3 (VI) | CE 2 (VII) | 4 (VIII) | 5 (IX) |
| Carbon efficiency | % | 76.8 | 82.0 | 88.2 | 96.6 | 68.4 | 84.7 | 85.9 |
| CO₂ emissions | t/yr | 691,000 | 504,000 | 12,000 | 10,000 | 1,056,000 | 13,000 | 13,000 |
| Net specific energy consumption | GJ/t | 35.8 | 33.9 | 29.9 | 30.2 | 40.1 | 30.8 | 31.2 |

## Claims

1. A method of producing methanol in a methanol synthesis plant, the method comprising:
(a) subjecting a feed comprising methane, ethane, propane, butane, LPG, naphtha, biogas, or a combination thereof to reforming to produce a reformer product comprising carbon monoxide and hydrogen, wherein a reforming temperature is maintained by a net heat input Q1;
(b) cooling the reformer product to produce a cooled reformer product by effecting a net heat removal Q2;
(c) shifting the reformer product to produce a shifted reformer product;
(d) cooling the shifted product by effecting a net heat removal Q3;
(e) cooling and condensing water from the reformer product by effecting a net heat removal Q4 to provide a purified reformer product;
(f) compressing the purified reformer product;
(g) heating the compressed, purified reformer product by a net heat input Q5 to provide a methanol synthesis feed;
(h) producing a product comprising methanol from the methanol synthesis feed;
(i) cooling the product comprising methanol by a net heat removal Q6 to remove a crude methanol stream and provide a recycle gas stream comprising carbon monoxide and hydrogen;
(j) compressing the recycle gas stream via a recycle compressor;
(k) purging via a purge gas system, wherein purging may be effected by a net heat input or removal Q7;
(l) purifying the methanol in the crude methanol product by removing light ends, heavy byproducts, water, or a combination thereof from the crude methanol stream, wherein removing the light ends, the heavy byproducts, the water, or the combination thereof requires a net heat input or removal Q8; or
(m) a combination thereof,
wherein, relative to a conventional method of producing methanol, more of the energy required for a net energy input or removal for a given step (a), (b), (c), (d), (e), (f), (g), (h), (i), (j), (k), (1), or a combination thereof (m), throughout the entire methanol synthesis plant, the compressing, or a combination thereof is provided by a non-carbon based energy source, a renewable energy source, and/or electricity.

2. The method of claim 1, wherein the non-carbon based energy source comprises electricity.

3. A method of producing methanol in a methanol synthesis plant, the method comprising:
(a) subjecting a feed comprising methane, ethane, propane, butane, LPG, naphtha, biogas, or a combination thereof to reforming to produce a reformer product comprising carbon monoxide and hydrogen, wherein a reforming temperature is maintained by a net heat input Q1;
(b) cooling the reformer product to produce a cooled reformer product by effecting a net heat removal Q2;
(c) shifting the reformer product to produce a shifted reformer product;
(d) cooling the shifted product by effecting a net heat removal Q3;
(e) cooling and condensing water from the reformer product by effecting a net heat removal Q4 to provide a purified reformer product;
(f) compressing the purified reformer product;
(g) heating the compressed, purified reformer product by a net heat input Q5 to provide a methanol synthesis feed;
(h) producing a product comprising methanol from the methanol synthesis feed;
(i) cooling the product comprising methanol by a net heat removal Q6 to remove a crude methanol stream and provide a recycle gas stream comprising carbon monoxide and hydrogen;
(j) compressing the recycle gas stream via a recycle compressor;
(k) purging via a purge gas system, wherein purging is effected by a net heat input or removal Q7;
(l) purifying the methanol in the crude methanol product by removing light ends, heavy byproducts, water, or a combination thereof from the crude methanol stream, wherein removing the light ends, the heavy byproducts, the water, or the combination thereof requires a net heat input or removal Q8; or
(m) a combination thereof,
wherein a majority of the energy required for a net energy input or removal for a given step (a), (b), (c), (d), (e), (f), (g), (h), (i), (j), (k), (1), or a combination thereof (m), throughout the entire methanol synthesis plant, the compressing, or a combination thereof is provided by a non-carbon based energy source, a renewable energy source, and/or electricity.

4. The method of claim 2 or 3, wherein the electricity is produced from a renewable energy source.

5. The method of claim 4, wherein the renewable energy source comprises wind, solar, geothermal, hydroelectric, nuclear, tide, wave, or a combination thereof.

6. The method of claim 1, 2 or 3, wherein maintaining the reforming temperature is effected without combusting a fuel, a fossil fuel, a carbon-based fuel, or a combination thereof externally to the feed.

7. The method of claim 6, wherein the non-carbon based energy source comprises or the electricity is produced via an intermittent energy source (IES), and further comprising maintaining the reforming temperature without combusting a fuel, a fossil fuel, a carbon-based fuel, or a combination thereof externally to the feed when the IES is available, and maintaining the reforming temperature via a stored supply of energy from the IES or by combusting a fuel or a carbon-based fuel when the IES is not available.

8. The method of claim 2 or 3, wherein the reforming is carried out in one or more reformers that are maintained at the reforming temperature via heating from electricity or renewable electricity and including associated convective, radiant or other heat transfer means.

9. The method of claim 8, wherein the heating from electricity or renewable electricity is resistive or inductive heating.

10. The method of claim 2 or 3, wherein:
other than the production of steam for use in a steam methane reformer and/or a pre-reformer, steam is not utilized as a primary energy transfer medium.

11. The method of claim 10, wherein:
a majority, some, or all of the external energy input for generating steam utilized in a steam methane reformer, a pre-reformer, one or more, a majority, or all steam turbines of the plant, or a combination thereof is electricity.

12. The method of claim 2 or 3, wherein heat removal in one or more or all steps (a)-(1) does not comprise the production of steam for use in a steam turbine.

13. The method of claim 2 or 3, wherein compressing the dry syngas or the purified reformer product, compressing the recycle gas stream, compressing the feed stream for introduction into one or more reformers, or a combination thereof comprises compressing with a compressor driven by an electric motor, an electrically-driven turbine, or a turbine driven by steam produced electrically in at least one, most, or all compressors utilized.

14. The method of claim 2 or 3, wherein the heat obtained from cooling the reformer product (e.g., via heat removal Q2, Q3, and/or Q4) is used to preheat the feed (e.g., used to provide a first portion of the heat input Q1).

15. The method of claim 14, wherein a second portion of the heat needed for heat input Q1 is provided by electrical heating, superheated steam, internal oxidation obtained by adding a small amount of oxygen or air, or a combination thereof.
